# EUROPEAN PATENT APPLICATION

(11) **EP 4 693 304 A1**
(43) Date of publication of application: **11.02.2026**
(21) Application number: 24778578.5
(22) Date of filing: 19.01.2024
(51) Int. Cl.: G16C 20/30, G06F 16/9038

(54) **INFORMATION PROCESSING DEVICE, INFORMATION PROCESSING METHOD, AND PROGRAM**

(30) Priority: 31.03.2023 JP 2023059423
(71) Applicant: FUJIFILM Corporation, Tokyo 106-8620 (JP)
(72) Inventor: TATESHITA, Masakazu, Ashigarakami-gun, Kanagawa 258-8577 (JP); HIKIDA, Yasushi, Ashigarakami-gun, Kanagawa 258-8577 (JP); MURAKAMI, Ryoichi, Ashigarakami-gun, Kanagawa 258-8577 (JP); SUGIYAMA, Satoshi, Ashigarakami-gun, Kanagawa 258-8577 (JP)
(74) Representative: HGF
(86) International application number: PCT/JP2024/001437
(87) International publication number: WO 2024/202432

(57) **Abstract**

An information processing apparatus includes a processor, in which the processor is configured to generate a display image that shows a prediction result of metabolic reactions occurring in vivo from a test substance and metabolites generated by the metabolic reactions and an evaluation result of toxicity of at least one compound in a compound group composed of the test substance and the metabolites, and that includes a determination flow indicating a toxicity determination procedure, which is used in an evaluation of the toxicity, has at least one determination step, and has a path that branches according to a determination result of the determination step, in which, in a case where the compound is selected in the display image, a path that leads to the evaluation result of the compound among a plurality of paths is shown in a distinguishable manner from the other paths, and execute control of outputting the generated display image.

## Description

### BACKGROUND OF THE INVENTION

### 1. Field of the Invention

The technology of the present disclosure relates to an information processing apparatus, an information processing method, and a program.

### 2. Description of the Related Art

In recent years, in the field of handling chemical substances, a toxicity evaluation using a computer-based in silico method has become common. In the toxicity evaluation of chemical substances, it is necessary to evaluate not only a test substance to be tested but also toxicity of its metabolites in order to evaluate its toxicity to a living body.

JP2013-522649A discloses a method for screening compounds including pharmaceuticals, lead and candidate drug compounds, and other chemical substances, using a biomarker profile of a metabolite and human stem cell-like cells (hSLCs) or lineage-specific cells produced from the human stem cell-like cells. The method according to the embodiment of the present invention is useful for testing toxicity, particularly developmental toxicity, and detecting teratogenic effects of such compounds. In particular, it is described to disclose a developmental toxicity model having higher predictivity based on an in vitro method that uses both hSLCs and metabolomics in order to discover a biomarker for developmental toxicity.

WO2011/055820A discloses a support device comprising: a measurement data acquisition unit that acquires a plurality of measurement data obtained under different conditions for each of a plurality of types of metabolites and/or metabolic enzymes; a pathway map data acquisition unit that acquires data representing a metabolic pathway map within a living body; an association strength calculation unit that calculates strength of association between the metabolites and/or metabolic enzymes and each metabolic pathway based on the measurement data acquired by the measurement data acquisition unit; a display unit; and a display content determination unit that determines a content to be displayed on the display unit. In the support device, the display content determination unit can cause the display unit to display a content representing two or more metabolic pathways such that the strength of the association is distinguishable, the support device further comprises a pathway selection input receiving unit that receives a selection input of one of the metabolic pathways from a user in a case where the content representing the two or more metabolic pathways is displayed on the display unit, and the display content determination unit causes the display unit to display the metabolic pathway map for the metabolic pathway selected by the selection input received by the pathway selection input receiving unit, and determines the content to be displayed on the display unit such that an increase or decrease in measurement values indicated by the measurement value data for the metabolites and/or metabolic enzymes present on the metabolic pathway map is distinguishable.

JP2004-93234A discloses a toxicity presence/absence determination system including: a storage unit that stores a simultaneous differential equation based on gene pathway information related to metabolism or a sequence of a toxic substance, a reaction rate coefficient of the simultaneous differential equation, a relationship between linear stability and Jacobian stability according to the reaction rate coefficient, and a determination of presence or absence of toxicity of the toxic substance in a value of the reaction rate coefficient; an input unit that inputs gene expression distribution data to the simultaneous differential equation; and a display unit that obtains the reaction rate coefficient by inputting the gene expression distribution data to the simultaneous differential equation and that displays the determination of the presence or absence of the toxicity of the toxic substance.

### SUMMARY OF THE INVENTION

However, in the technologies disclosed in JP2013-522649A, WO2011/055820A, and JP2004-93234A, a display method of a prediction result of a metabolic reaction and an evaluation result of toxicity is not considered. Therefore, in a determination flow used for an evaluation of toxicity to a compound involved in the metabolic reaction, there is room for improvement in displaying a path that leads to the evaluation result of the toxicity.

One embodiment according to the technology of the present disclosure provides an information processing apparatus, an information processing method, and a program that, in evaluating toxicity of a compound, make it easier to understand which evaluation path has been followed for the toxicity evaluation and that support a user in evaluating the toxicity of the compound.

A first aspect according to the technology of the present disclosure is an information processing apparatus comprising: a processor, in which the processor is configured to generate a display image that shows a prediction result of metabolic reactions occurring in vivo from a test substance and metabolites generated by the metabolic reactions and an evaluation result of toxicity of at least one compound in a compound group composed of the test substance and the metabolites, and that includes a determination flow indicating a toxicity determination procedure, which is used in an evaluation of the toxicity, has at least one determination step, and has a path that branches according to a determination result of the determination step, in which, in a case where the compound is selected in the display image, a path that leads to the evaluation result of the compound among a plurality of paths is shown in a distinguishable manner from the other paths, and execute control of outputting the generated display image.

A second aspect according to the technology of the present disclosure is the information processing apparatus according to the first aspect, in which the display image includes a first display region in which an explanation regarding the determination flow is displayed, and an explanation of a determination content is displayed in the first display region for each determination step included in the determination flow.

A third aspect according to the technology of the present disclosure is the information processing apparatus according to the second aspect, in which, in the first display region, details of the determination result are displayed for each determination step included in the determination flow.

A fourth aspect according to the technology of the present disclosure is the information processing apparatus according to the third aspect, in which the details of the determination result include a numerical value used in a comparison with a threshold value in a case where the determination step involves the comparison with the threshold value, the numerical value indicating a characteristic of a molecular structure of the metabolite.

A fifth aspect according to the technology of the present disclosure is the information processing apparatus according to the fourth aspect, in which the details of the determination result is switchable between display and non-display.

A sixth aspect according to the technology of the present disclosure is the information processing apparatus according to the first aspect, in which, in the display image, the evaluation result of the compound is displayed in a distinguishable manner for each compound in the prediction result.

A seventh aspect according to the technology of the present disclosure is the information processing apparatus according to the first aspect, in which, in the display image, the compound selected for displaying the determination flow is displayed in a distinguishable manner in the prediction result.

An eighth aspect according to the technology of the present disclosure is the information processing apparatus according to the first aspect, in which, in the display image, as the prediction result, a metabolic reaction pathway that is systematically shown using the metabolic reactions occurring in a chain starting from the test substance and the resulting metabolites is shown.

A ninth aspect according to the technology of the present disclosure is the information processing apparatus according to the eighth aspect, in which the metabolic reaction pathway shown in the display image is a tree diagram shown by compound data representing each compound of the test substance and the metabolites and connecting lines connecting items of the compound data before and after the metabolic reaction.

A tenth aspect according to the technology of the present disclosure is the information processing apparatus according to the ninth aspect, in which a name of the metabolic reaction is displayed in association with the connecting line.

An eleventh aspect according to the technology of the present disclosure is an information processing method comprising: generating a display image that shows a prediction result of metabolic reactions occurring in vivo from a test substance and metabolites generated by the metabolic reactions and an evaluation result of toxicity of at least one compound in a compound group composed of the test substance and the metabolites, and that includes a determination flow indicating a toxicity determination procedure, which is used in an evaluation of the toxicity, has at least one determination step, and has a path that branches according to a determination result of the determination step, in which, in a case where the compound is selected in the display image, a path that leads to the evaluation result of the compound among a plurality of paths is shown in a distinguishable manner from the other paths; and executing control of outputting the generated display image.

A twelfth aspect according to the technology of the present disclosure is a program for causing a computer to execute a process comprising: generating a display image that shows a prediction result of metabolic reactions occurring in vivo from a test substance and metabolites generated by the metabolic reactions and an evaluation result of toxicity of at least one compound in a compound group composed of the test substance and the metabolites, and that includes a determination flow indicating a toxicity determination procedure, which is used in an evaluation of the toxicity, has at least one determination step, and has a path that branches according to a determination result of the determination step, in which, in a case where the compound is selected in the display image, a path that leads to the evaluation result of the compound among a plurality of paths is shown in a distinguishable manner from the other paths; and executing control of outputting the generated display image.

The technology of the present disclosure provides an information processing apparatus, an information processing method, and a program that, in evaluating toxicity of a compound, make it easier to understand which evaluation path has been followed for the toxicity evaluation and that support a user in evaluating the toxicity of the compound.

### BRIEF DESCRIPTION OF THE DRAWINGS

FIG. 1 is a conceptual diagram showing a schematic configuration example of an information processing system.
FIG. 2 is a block diagram showing an example of a hardware configuration of an electrical system of a server.
FIG. 3 is a conceptual diagram showing an example of a usage aspect of the information processing system.
FIG. 4 is a functional block diagram showing an example of main functions of a processor of the server.
FIG. 5 is a conceptual diagram showing an example of processing contents of a toxicity evaluation unit.
FIG. 6 is a functional block diagram showing an example of main functions of the processor of the server.
FIG. 7 is a conceptual diagram showing an example of processing contents of an image generation unit.
FIG. 8 is a conceptual diagram showing an example of an aspect in which a part of a display image is displayed on a display device.
FIG. 9 is a conceptual diagram showing an example of an aspect in which a part of a display image is displayed on a display device.
FIG. 10 is a conceptual diagram showing an example of an aspect in which display of details of a display image is switched.
FIG. 11 is a flowchart showing an example of a flow of server control processing.
FIG. 12 is a functional block diagram showing an example of main functions of the processor of the server.
FIG. 13 is a conceptual diagram showing an example of an aspect in which a display image is displayed on the display device.
FIG. 14 is a conceptual diagram showing an example of an aspect in which a display image is displayed on the display device.
FIG. 15 is a conceptual diagram showing an example of an aspect in which a display image is displayed on the display device.
FIG. 16 is a block diagram showing an example of a hardware configuration of an electrical system of a client terminal.
FIG. 17 is a functional block diagram showing an example of main functions of a processor of the client terminal.
FIG. 18 is a functional block diagram showing an example of main functions of the processor of the client terminal.

### DESCRIPTION OF THE PREFERRED EMBODIMENTS

An example of embodiments of an information processing apparatus, an information processing method, and a program according to the technology of the present disclosure will be described with reference to the accompanying drawings.

### [First Embodiment]

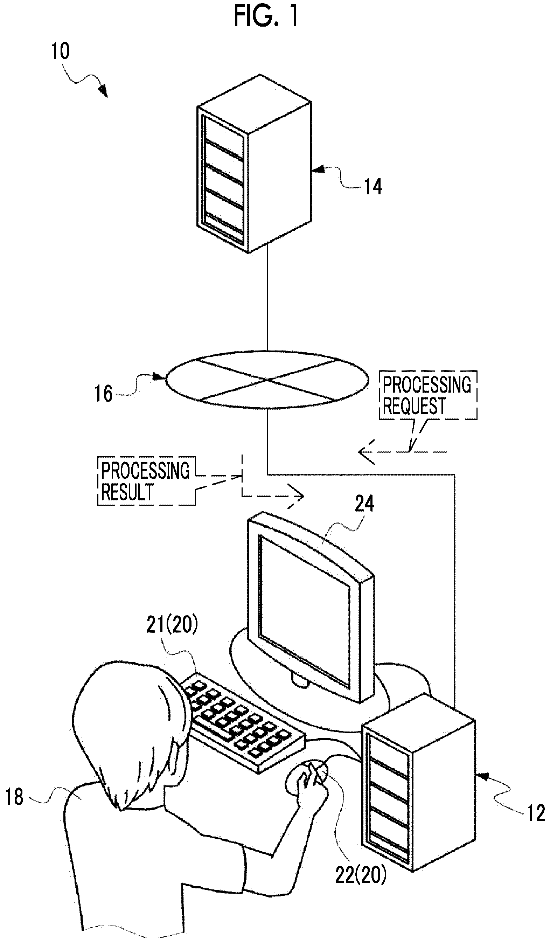

As shown in FIG. 1 as an example, an information processing system 10 comprises a client terminal 12 and a server 14. In the information processing system 10, the client terminal 12 and the server 14 are communicably connected to each other via a network 16.

The information processing system 10 is used, for example, for a prediction of a reaction (for example, a metabolic reaction in a body) related to a chemical substance and/or an evaluation of a characteristic of a chemical substance (for example, an evaluation of the presence or absence of toxicity to a human body). A user 18 (for example, a researcher) can use the information processing system 10 to predict and evaluate experimental results using computer simulations (that is, an evaluation using in silico method). For example, for a newly developed or under-development chemical substance, it is possible to obtain an evaluation result of toxicity without performing a toxicity evaluation test (that is, an in vitro or in vivo toxicity evaluation test) using an actual chemical substance.

The client terminal 12 is a terminal used by the user 18. The server 14 receives a processing request from the client terminal 12 via the network 16 and provides a service corresponding to the request to the client terminal 12 that has made the request via the network 16. For example, in a case where the server 14 receives a request to execute processing related to a reaction prediction and a characteristic evaluation of a chemical substance as the processing request, the server 14 transmits a prediction result and an evaluation result to the client terminal 12. In the present embodiment, the server 14 is an example of an "information processing apparatus" according to the technology of the present disclosure.

For example, the server 14 is realized by a mainframe, but this is merely an example. For example, the server may be realized by cloud computing, or may be realized by network computing such as fog computing, edge computing, or grid computing. Here, the server 14 is exemplified as an example of a device provided outside the client terminal 12, but this is merely an example, and at least one personal computer or the like may be used instead of the server 14.

The network 16 is configured by, for example, at least one of a wide area network (WAN) or a local area network (LAN). Further, a connection method between the client terminal 12 and the network 16 and a connection method between the server 14 and the network 16 may be a wireless communication method or a wired communication method. The network 16 establishes communication between the client terminal 12 and the server 14, and transmits and receives various types of information between the client terminal 12 and the server 14.

A reception device 20 is connected to the client terminal 12. The reception device 20 receives an instruction from the user 18. The reception device 20 includes a keyboard 21, a mouse 22, and the like. The keyboard 21 and the mouse 22 shown in FIG. 1 are merely an example. As the reception device 20, any one of the keyboard 21 or the mouse 22 may be provided. In addition, as the reception device 20, for example, at least one of a proximity input device that receives a proximity input, a voice input device that receives a voice input, or a gesture input device that receives a gesture input may be applied instead of the keyboard 21 and/or the mouse 22. The proximity input device is, for example, a touch panel or a tablet.

A display device 24 is connected to the client terminal 12. Examples of the display device 24 include an electro-luminescence (EL) display and a liquid crystal display. The display device 24 displays various types of information (for example, image and text) under the control of the client terminal 12.

Here, a personal computer is described as an example of the client terminal 12, but this is merely an example. As the client terminal 12, a mobile terminal, such as a smartphone and a tablet terminal, may be used. Here, although an example in which one client terminal 12 is connected to one server 14 via the network 16 has been described, this is merely an example. It is needless to say that the information processing system 10 may include a plurality of client terminals 12 and a plurality of servers 14.

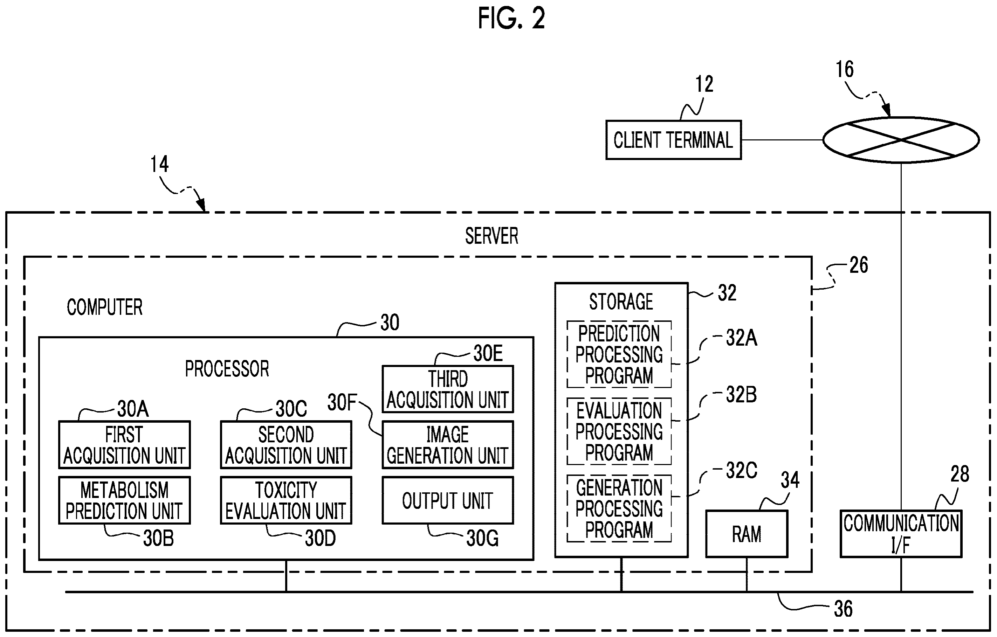

As shown in FIG. 2 as an example, the server 14 comprises a computer 26, a communication interface (I/F) 28, and a bus 36. The computer 26 comprises a processor 30, a storage 32, and a random access memory (RAM) 34. The processor 30, the storage 32, the RAM 34, and the communication I/F 28 are connected to the bus 36. The computer 26 is an example of a "computer" according to the technology of the present disclosure, and the processor 30 is an example of a "processor" according to the technology of the present disclosure.

A memory is connected to the processor 30. The memory includes the storage 32 and the RAM 34. The processor 30 includes, for example, a central processing unit (CPU) and a graphics processing unit (GPU). The GPU operates under the control of the CPU, and is responsible for executing processing related to an image.

The storage 32 is a non-volatile storage device that stores various programs, various parameters, and the like. Examples of the storage 32 include a flash memory (for example, an electrically erasable and programmable read only memory (EEPROM) and a solid state drive (SSD)), and/or a hard disk drive (HDD). The flash memory and the HDD are merely examples, and at least one of the flash memory, the HDD, a magnetoresistive memory, or a ferroelectric memory may be used as the storage 32.

The RAM 34 is a memory that transitorily stores information, and is used as a work memory by the processor 30. Examples of the RAM 34 include a dynamic random access memory (DRAM) or a static random access memory (SRAM).

The communication I/F 28 is connected to the network 16. The communication I/F 28 is responsible for transmitting and receiving information to and from an external communication device (for example, the client terminal 12) via the network 16. For example, the communication I/F 28 transmits information in response to a request from the processor 30 to the external communication device via the network 16. In addition, the communication I/F 28 receives the information transmitted from the external communication device, and outputs the received information to the processor 30 via the bus 36.

A prediction processing program 32A is stored in the storage 32. The prediction processing program 32A is a program that provides a prediction simulation related to metabolism of a chemical substance. The processor 30 reads out the prediction processing program 32A from the storage 32 and executes the read-out prediction processing program 32A on the RAM 34 to perform metabolism prediction processing. The metabolism prediction processing is realized by the processor 30 operating as a first acquisition unit 30A and a metabolism prediction unit 30B.

In addition, an evaluation processing program 32B is stored in the storage 32. The evaluation processing program 32B is a program that provides an evaluation simulation of toxicity of a chemical substance. The processor 30 reads out the evaluation processing program 32B from the storage 32 and executes the read-out evaluation processing program 32B on the RAM 34 to perform toxicity evaluation processing. The toxicity evaluation processing is realized by the processor 30 operating as a second acquisition unit 30C and a toxicity evaluation unit 30D.

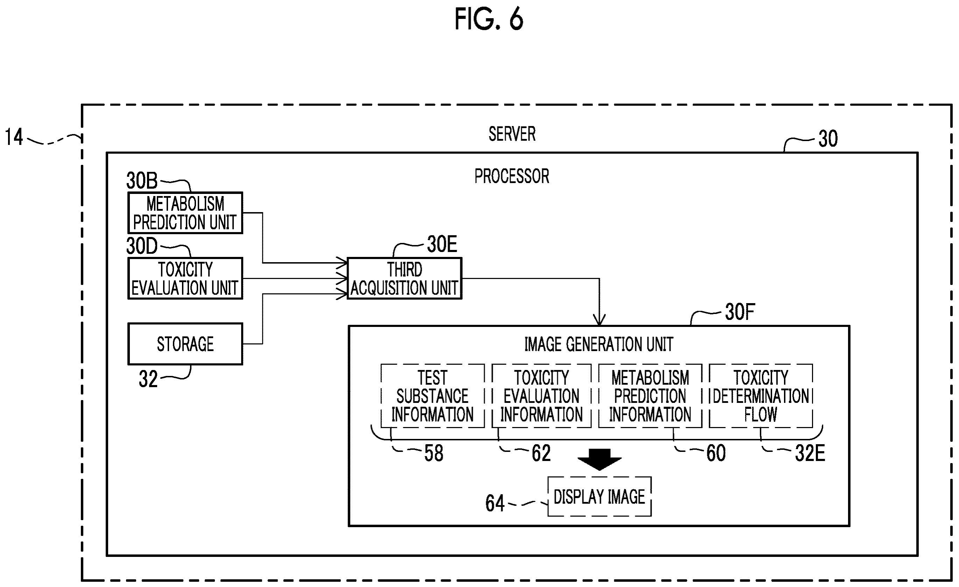

In addition, a generation processing program 32C is stored in the storage 32. The generation processing program 32C is a program that generates a display image 64 (see FIG. 6 and the like) to be output to the client terminal 12. The processor 30 reads out the generation processing program 32C from the storage 32 and executes the read-out generation processing program 32C on the RAM 34 to perform image generation processing. The image generation processing is realized by the processor 30 operating as a third acquisition unit 30E, an image generation unit 30F, and an output unit 30G. The generation processing program 32C is an example of a "program" according to the technology of the present disclosure.

Here, a case where the user 18 uses the information processing system 10 to make a prediction regarding metabolism in a body (hereinafter, also simply referred to as a "metabolism prediction") and an evaluation of toxicity to a human body (hereinafter, also simply referred to as a "toxicity evaluation") for a newly developed chemical substance A will be considered. Here, the toxicity refers to toxicity that the user 18 is interested in as an evaluation item, and examples thereof include mutagenicity, sensitization, and irritation. The mutagenicity refers to a property of causing an irreversible change in genetic information (a base sequence of deoxyribonucleic acid (DNA) or a structure or number of chromosomes) of an organism. The sensitization refers to a property of causing an allergic reaction upon exposure to a chemical substance. The irritation refers to a property in which a chemical substance or the like gives a stimulus (for example, pain or a burning sensation) to the tactile sense or the like.

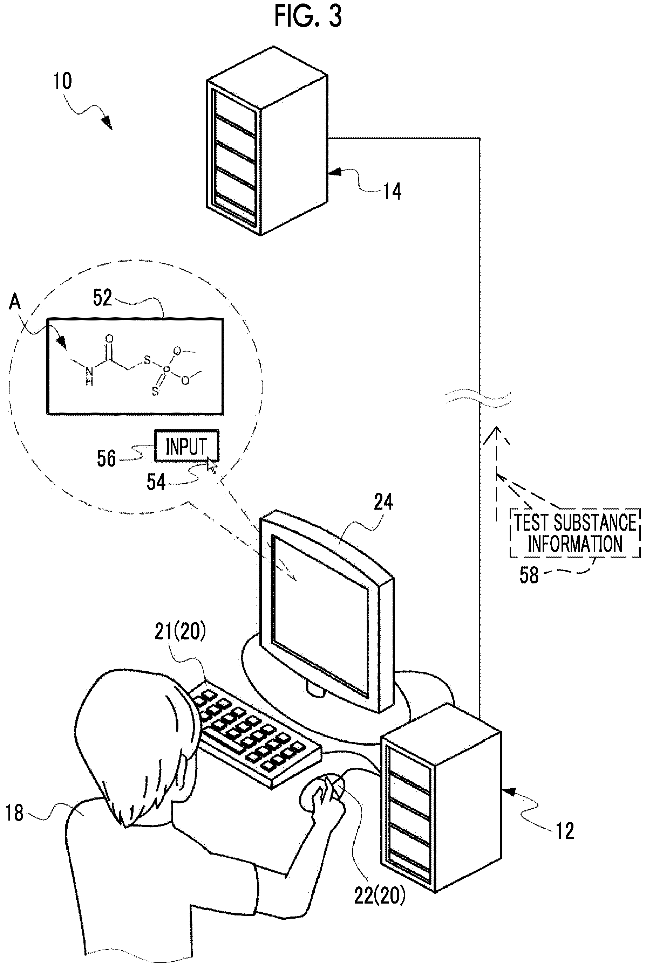

In a case of evaluating the toxicity, as shown in FIG. 3 as an example, the user 18 inputs data indicating a chemical substance A (hereinafter, also simply referred to as a "test substance A") that is a target of the metabolism prediction and the toxicity evaluation to the client terminal 12 via the reception device 20. Examples of the test substance include a low-molecular-weight compound (for example, a compound having a molecular weight of less than 1000), a sugar, a peptide, a protein, and a nucleic acid. The test substance A is an example of a "test substance" according to the technology of the present disclosure.

In the example shown in FIG. 3, a screen for selecting a test substance is displayed on a screen of the display device 24, and the user 18 performs a selection operation on the selection screen to select, for example, the test substance A. On the selection screen, for example, a test substance graph 52 is displayed, which shows a chemical structure of the test substance A as a compound graph (that is, a data structure in which atoms are nodes and bonds are edges). The user 18 clicks an input soft key 56 by operating a pointer 54 displayed on the screen of the display device 24 via the mouse 22. As a result, test substance information 58 is transmitted from the client terminal 12 to the server 14, and a processing request is made regarding the metabolism prediction and the toxicity evaluation for the test substance A.

Here, the test substance information 58 is information for specifying the chemical structure of the test substance A. The test substance graph 52 is, for example, image data, and the test substance information 58 is information composed of text information or numerical information representing a chemical structure of the test substance. The display device 24 reads out the test substance information 58 corresponding to the selected test substance graph 52 with reference to table data or the like in which a correspondence relationship between the test substance graph 52 and the test substance information 58 is recorded.

Here, although an example in which the compound graph is input as the data indicating the test substance A has been described, this is merely an example. The data input to the client terminal 12 need only be data for specifying the test substance A, and may be the test substance information 58. More specifically, the test substance information 58 is text information describing a chemical structure such as a structural formula, a compositional formula, and an amino acid sequence, or numerical information obtained by converting such text information into a numerical value. In addition, the data indicating the test substance A may be a chemical abstract service (CAS) number or a name of a chemical substance.

In addition, there may be a plurality of test substances A. In this case, for example, the user 18 may input data in which the plurality of test substances A are listed.

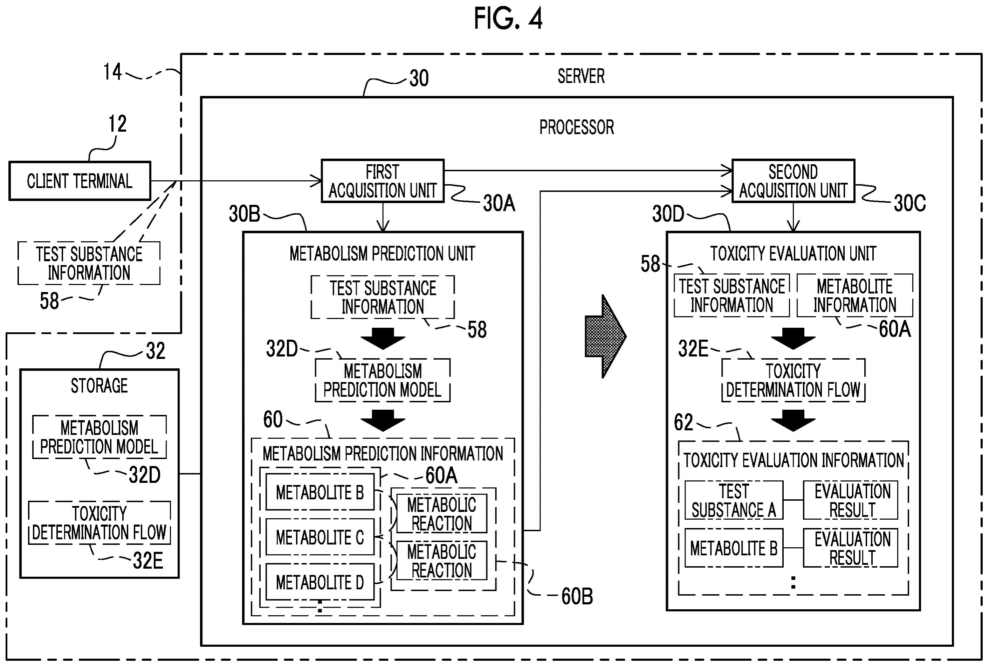

As shown in FIG. 4 as an example, the test substance information 58 is output from the client terminal 12 to the server 14. In the processor 30 of the server 14, the metabolism prediction processing is executed. The metabolism prediction processing is processing of predicting a metabolic reaction pathway of a test substance that occurs in a case where the test substance is subjected to a toxicity evaluation test and metabolites generated in the metabolic reaction pathway, and outputting the metabolic reaction pathway and the metabolites as a prediction result. Hereinafter, the test substance and the metabolite may be collectively referred to as simply "compound".

In the metabolism prediction processing, first, the first acquisition unit 30A acquires the test substance information 58. The first acquisition unit 30A outputs the test substance information 58 to the metabolism prediction unit 30B. The metabolism prediction unit 30B makes a prediction regarding the metabolism of the test substance A in the body, which is indicated by the test substance information 58. Here, the prediction regarding the metabolism includes a prediction of metabolic reactions that occur in a chain starting from the test substance A and a prediction of metabolites, which are compounds generated by the metabolic reactions.

Specifically, the metabolism prediction unit 30B acquires a metabolism prediction model 32D from the storage 32. The metabolism prediction model 32D is a model including rule data for predicting the metabolic reaction pathway and the metabolite. The processor 30 executes the metabolism prediction processing based on the test substance information 58 and the rule data. The rule data is data representing a rule in which a metabolic reaction proceeds. The rule data includes, for example, information on a metabolic reaction of a compound, that is, information on what kind of metabolic reaction a compound having what kind of structure generates and what kind of metabolite is generated. The processor 30 predicts the metabolic reaction and the metabolite while collating the test substance information 58 with the rule data. The metabolic reaction occurs not only in the test substance but also in the metabolite. Therefore, the metabolic reactions occur in a chain starting from the test substance. In a case where a metabolite is predicted by a metabolic reaction occurring in the test substance, the processor 30 also predicts a metabolic reaction occurring in the predicted metabolite. As described above, the processor 30 predicts metabolic reactions that occur in a chain in each compound of the test substance and the metabolite, and records the metabolic reactions as a metabolic reaction pathway.

Here, although an example of a form in which the prediction of the metabolic reaction and the metabolite is performed using the rule-based metabolism prediction model 32D using the rule data has been described, this is merely an example. The metabolism prediction model 32D may be, for example, a trained model for predicting a metabolic reaction using an artificial intelligence (AI) method. Such a trained model realizes a function of predicting a metabolic reaction, for example, by training a neural network through machine learning using training data. An example of training data is a data set obtained from results of past experiments, in which information for specifying a test substance is used as example data, and information for specifying a metabolite and information for specifying a metabolic reaction are used as correct answer data.

Then, the metabolism prediction unit 30B inputs the test substance information 58 to the metabolism prediction model 32D. The metabolism prediction model 32D outputs metabolism prediction information 60 corresponding to the input test substance information 58. The metabolism prediction unit 30B acquires the metabolism prediction information 60 output from the metabolism prediction model 32D. The metabolism prediction information 60 includes metabolic reaction information 60B that is information for specifying the predicted metabolic reaction, and metabolite information 60A that is information for specifying the metabolite generated by the metabolic reaction. The metabolite information 60A is information for specifying a chemical structure of a metabolite, which is a compound, as with the test substance information 58.

The first acquisition unit 30A outputs the test substance information 58 to the second acquisition unit 30C, and the metabolism prediction unit 30B outputs the metabolism prediction information 60 to the second acquisition unit 30C. After the metabolism prediction processing is executed by the processor 30, the processor 30 proceeds to the toxicity evaluation processing.

The toxicity evaluation processing is processing of evaluating toxicity of at least one compound selected from the group consisting of a test substance and metabolites. In the toxicity evaluation processing, the second acquisition unit 30C outputs the test substance information 58 and the metabolism prediction information 60 to the toxicity evaluation unit 30D. The toxicity evaluation unit 30D evaluates the toxicity of the test substance A indicated by the test substance information 58 and toxicity of the metabolite indicated by the metabolite information 60A.

Specifically, the toxicity evaluation unit 30D acquires a toxicity determination flow 32E from the storage 32. The toxicity determination flow 32E is described in the evaluation processing program 32B, and is schematically shown in a form of being stored in the storage 32 in FIG. 4. The toxicity determination flow 32E is a determination flow used for evaluating the toxicity. The toxicity determination flow 32E has at least one determination step, and has a route that branches according to a determination result in the determination step. Determination items in each determination step are predetermined. Examples of the determination item include whether or not a compound disappears through metabolism, whether or not a compound permeates a cell membrane, and whether or not a compound corresponds to a structural-alert rule. The structural-alert rule is a regulation related to a chemical structure, and is a rule for specifying a partial structure that may have toxicity of interest to the user.

The toxicity evaluation unit 30D inputs the test substance information 58 and the metabolite information 60A to the toxicity determination flow 32E. In the toxicity determination flow 32E, a determination regarding the toxicity evaluation is executed for information indicating any compound of the test substance or the metabolite in each determination step. The toxicity determination flow 32E is an example of a "determination flow" according to the technology of the present disclosure. In addition, steps ST1 to ST5 described below are examples of a "determination step" according to the technology of the present disclosure. Hereinafter, the test substance information 58 and the metabolite information 60A will be collectively referred to as compound information.

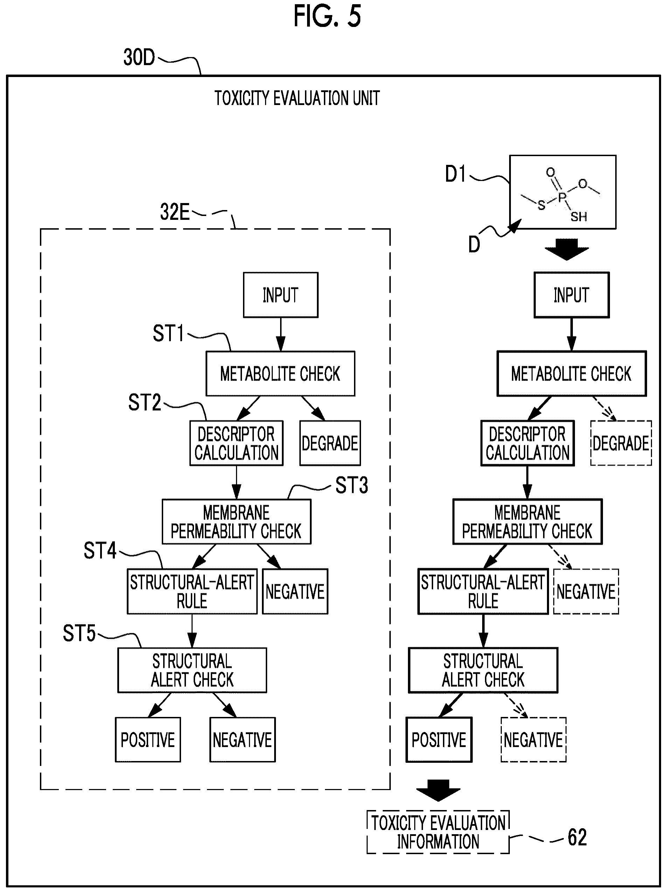

In the following description of the toxicity determination flow 32E, a metabolite D will be described as an example. As shown in FIG. 5 as an example, compound information corresponding to a compound graph D1 showing the metabolite D is input to the toxicity determination flow 32E. In the toxicity determination flow 32E, in step ST1, it is determined whether or not the metabolite D represented by the compound graph D1 is a compound that persists in the body without being metabolically degraded. In a case where the determination in step ST1 is affirmative, the toxicity evaluation for the metabolite D proceeds to step ST2. In a case where the determination in step ST1 is negative, as a result of the toxicity evaluation for the metabolite D, an evaluation result that the metabolite D is a compound (hereinafter, also simply referred to as a "disappearance compound") that is degraded and disappears in the body is output.

Here, in a metabolic reaction, a metabolic enzyme forms a complex with a compound that fits into an active site of the metabolic enzyme. The metabolic enzyme has an action of promoting a metabolic reaction by lowering the energy (that is, the activation energy) required for the compound to cause a chemical reaction. The term "disappearance compound" refers to a compound that, in a case of forming a complex with a metabolic enzyme, has lower activation energy than other metabolites, undergoes a chemical reaction more rapidly than other metabolites, and is converted into the next metabolite in a short period of time. Therefore, the disappearance compound is less likely to react with DNA in a cell, and the necessity of evaluating toxicity (that is, positive or negative evaluation) is low.

In step ST2, descriptor calculation is executed for the compound information corresponding to the compound graph D1. Examples of the descriptor include a molar mass or a LogP (a value obtained by taking the common logarithm of an octanol/water partition coefficient) of the metabolite D. After the process of step ST2 is executed, the toxicity evaluation proceeds to step ST3.

In step ST3, it is determined whether or not the compound can permeate the cell membrane based on a result of the descriptor calculation of the metabolite D executed in step ST2. The numerical value indicated by the descriptor is compared with a threshold value, and a determination is made as to whether or not the compound can permeate the cell membrane based on a comparison result. In a case where the determination in step ST3 is affirmative, the toxicity evaluation proceeds to step ST4. In a case where the determination in step ST3 is negative, a negative (that is, non-toxic) evaluation result is output as the result of the toxicity evaluation. This is because the compound does not permeate the cell membrane and is therefore considered to have little effect on the body.

In step ST4, the structural-alert rule is applied to the compound information corresponding to the compound graph D1. After the process of step ST4 is executed, the toxicity evaluation proceeds to step ST5.

In step ST5, it is determined whether or not a molecular structure indicated by the compound information corresponding to the compound graph D1 includes a structural alert. In a case where the determination in step ST5 is negative, a negative (that is, non-toxic) evaluation result is output as the result of the toxicity evaluation. This is because the metabolite D does not have a structural alert and is therefore considered to have a low likelihood of exhibiting toxicity.

In a case where the determination in step ST5 is affirmative, a positive (that is, toxic) evaluation result is output as the result of the toxicity evaluation. In the example shown in FIG. 5, paths and determination steps taken in the toxicity determination flow 32E are shown as the toxicity evaluation for the metabolite D, and an example in which the metabolite D is finally evaluated as positive is shown. In addition, the toxicity evaluation information 62 includes information indicating the result of the toxicity evaluation as well as information indicating a path (hereinafter, also simply referred to as an "evaluation path") leading to the result of the toxicity evaluation in the toxicity determination flow 32E, information indicating an explanation of the determination item, and information indicating details of a determination result for each determination step.

As described above, the toxicity determination flow 32E outputs toxicity evaluation information 62 according to the input test substance information 58 and metabolite information 60A. In the toxicity evaluation information 62, the evaluation result is linked to each of the test substance A and its metabolites (see FIG. 4). In this way, the toxicity evaluation processing is executed.

Here, although an example of a form in which the toxicity evaluation processing is executed after the metabolism prediction processing is executed has been described, the technology of the present disclosure is not limited to this. The metabolism prediction processing and the toxicity evaluation processing may be executed in parallel. For example, during the metabolism prediction processing, the toxicity evaluation in the toxicity evaluation processing may be sequentially performed on each metabolite whose generation has been predicted.

As shown in FIG. 6 as an example, the metabolism prediction unit 30B outputs the metabolism prediction information 60 to the third acquisition unit 30E. In addition, the toxicity evaluation unit 30D outputs the test substance information 58 and the toxicity evaluation information 62 to the third acquisition unit 30E. The third acquisition unit 30E outputs the acquired test substance information 58, the acquired metabolism prediction information 60, and the acquired toxicity evaluation information 62 to the image generation unit 30F. In addition, the third acquisition unit 30E acquires the toxicity determination flow 32E from the storage 32 and outputs the toxicity determination flow 32E to the image generation unit 30F. The image generation unit 30F generates a display image 64 based on the test substance information 58, the metabolism prediction information 60, the toxicity evaluation information 62, and the toxicity determination flow 32E.

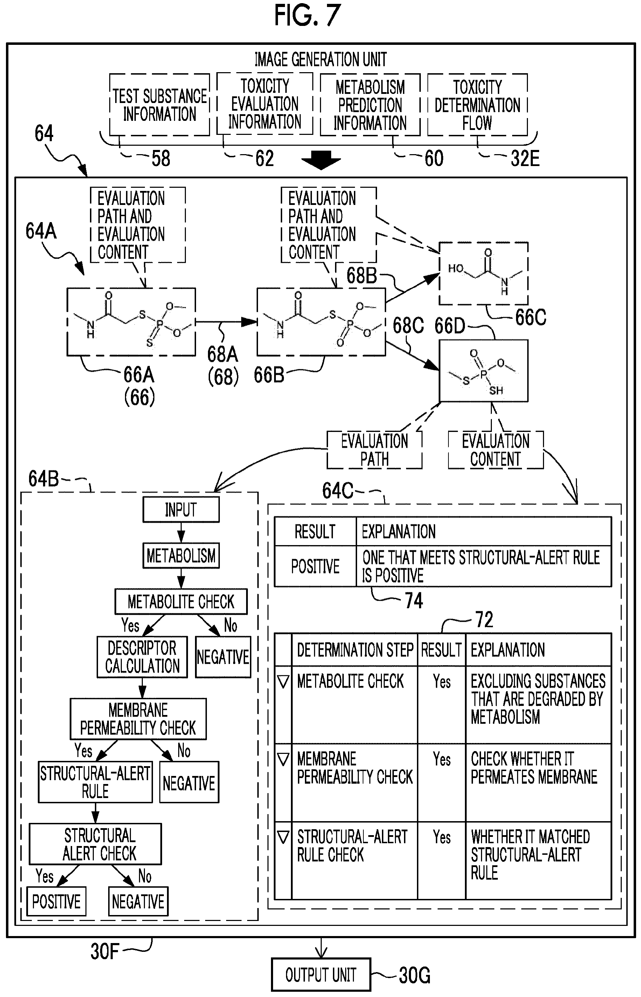

As shown in FIG. 7 as an example, the display image 64 is an image showing a result of a prediction regarding metabolism and a result of a toxicity evaluation for a compound. The display image 64 includes a metabolism prediction image 64A including a compound image 66 showing a compound graph of each of the test substance A and the metabolites B to D. The metabolism prediction image 64A is an image showing the evaluation result of the toxicity of the compound in a distinguishable manner in a metabolic reaction pathway that systematically shows the metabolic reactions that occur in a chain starting from the test substance and the resulting metabolites. In the example shown in FIG. 7, the metabolism prediction image 64A is a tree diagram showing the metabolic reaction pathway. The display image 64 is an example of a "display image" according to the technology of the present disclosure.

In the example shown in FIG. 7, the metabolism prediction image 64A includes a compound image 66 showing a compound graph of each of the test substance A and the metabolites B to D. In addition, the metabolism prediction image 64A includes a connecting line 68 that connects the compound images 66 before and after the metabolic reaction. The metabolites B to D are examples of a "metabolite" according to the technology of the present disclosure, the compound image 66 is an example of "compound data" according to the technology of the present disclosure, and the connecting line 68 is an example of a "connecting line" according to the technology of the present disclosure.

Specifically, a compound image 66A showing a compound graph of the test substance A is shown in a left portion of the metabolism prediction image 64A. In addition, a compound image 66B showing a compound graph of the metabolite B is shown in a central portion of the metabolism prediction image 64A. A connecting line 68A is shown between the compound image 66A and the compound image 66B.

In addition, a compound image 66C showing a compound graph of the metabolite C is shown in an upper right portion of the metabolism prediction image 64A. A connecting line 68B is shown between the compound image 66B and the compound image 66C. A compound image 66D showing a compound graph of the metabolite D is shown in a lower right portion of the metabolism prediction image 64A. A connecting line 68C is shown between the compound image 66B and the compound image 66D. As described above, in the display image 64, the metabolic reaction pathway that systematically shows the metabolic reactions that occur in a chain starting from the test substance A and the resulting metabolites B to D is shown as a tree diagram.

In the metabolism prediction image 64A, frame lines of the compound image 66A showing the test substance A and the compound image 66B showing the metabolite B are one-dot chain lines. This indicates that the test substance A and the metabolite B are disappearance compounds. In addition, in the metabolism prediction image 64A, a frame line of the compound image 66C showing the metabolite C is a dotted line. This indicates that the evaluation result of the toxicity of the metabolite C is "non-toxic". Further, in the metabolism prediction image 64A, a frame line of the compound image 66D showing the metabolite D is a solid line. This indicates that the evaluation result of the toxicity of the metabolite D is "toxic".

As described above, in the metabolism prediction image 64A, the evaluation result of the toxicity of the compound is shown in a distinguishable manner. In addition, the metabolism prediction image 64A shows the compound image 66B showing the metabolite B, which is a disappearance compound, is shown, and shows that the metabolite B is a disappearance compound in a distinguishable manner.

The display image 64 includes a flow image 64B showing the toxicity determination flow 32E. In the metabolism prediction image 64A, the compound indicated by the compound image 66 and the evaluation path in the toxicity determination flow 32E are associated with each other. As a result, in a case where any of the compound images 66 is selected in the metabolism prediction image 64A, the evaluation path of the compound indicated by the selected compound image 66 is displayed in the flow image 64B.

In addition, the display image 64 includes a flow explanation image 64C. The flow explanation image 64C is an image showing an explanation of the determination content of the toxicity determination flow 32E and details of the determination result. Here, the explanation of the determination content refers to an explanation of the determination performed in each determination step. In addition, the details of the determination result refer to details of the determination result of each determination step. The flow explanation image 64C is an example of a "first display region" according to the technology of the present disclosure.

The flow explanation image 64C includes a determination step explanation image 72 and a result explanation image 74. In the display image 64, the compound indicated by the compound image 66 of the metabolism prediction image 64A and the explanation of the determination in the determination step explanation image 72 are associated with each other. As a result, in a case where any of the compound images 66 is selected in the metabolism prediction image 64A, an explanation of the determination step used in the toxicity evaluation for the compound indicated by the selected compound image 66 is displayed in the determination step explanation image 72. The image generation unit 30F outputs the generated display image 64 to the output unit 30G.

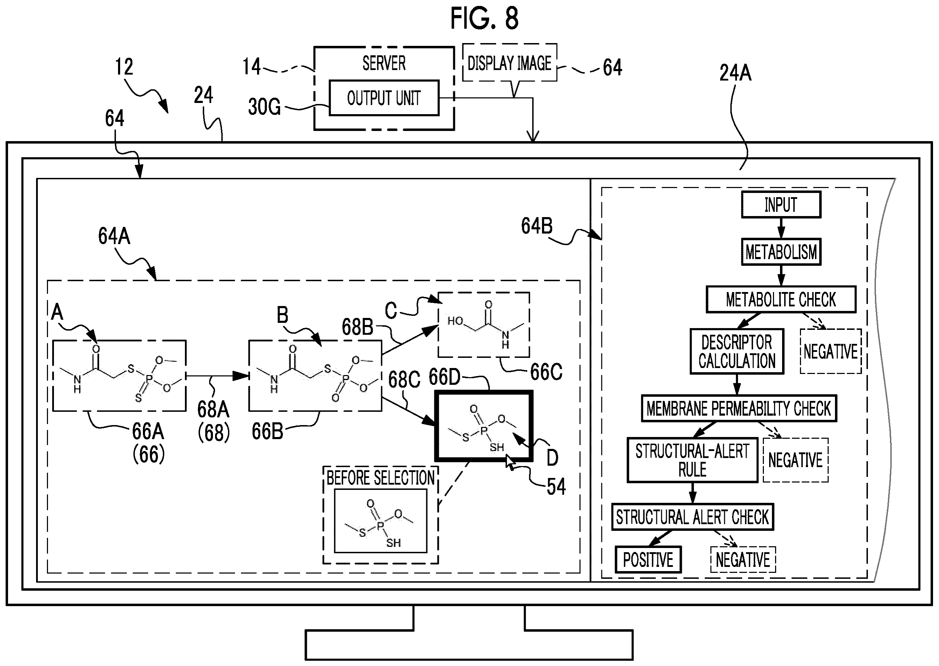

As shown in FIG. 8 as an example, the output unit 30G of the server 14 executes control of outputting the display image 64 to the client terminal 12. In other words, the server 14 transmits information indicating the display image 64 to the client terminal 12. On the display device 24 of the client terminal 12, a screen 24A including the display image 64 is displayed.

In the example shown in FIG. 8, the metabolism prediction image 64A is displayed at a left end portion of the display image 64. As described above, in the metabolism prediction image 64A, the metabolic reaction pathway that systematically shows the metabolic reactions that occur in a chain starting from the test substance A and the resulting metabolites B to D is shown as a tree diagram. In addition, in the metabolism prediction image 64A, the evaluation result of the toxicity of the compound is shown in a distinguishable manner. The user 18 can understand the result of the metabolism prediction for the test substance A and the result of the toxicity evaluation of the test substance A and the metabolites B to D by visually recognizing the metabolism prediction image 64A.

In addition, in the example shown in FIG. 8, in the display image 64, the flow image 64B is displayed on a right side of the metabolism prediction image 64A. In a case where the compound image 66 showing the compound is selected in the metabolism prediction image 64A, an evaluation path is displayed in the flow image 64B. In the example shown in FIG. 8, in a case where the compound image 66D is selected by the user 18 via the pointer 54, an evaluation path of the metabolite D indicated by the compound image 66D (here, a path leading to "positive") is displayed in the flow image 64B. In addition, in the flow image 64B, the evaluation path is shown to be distinguishable from other paths in the toxicity determination flow 32E. In the example shown in FIG. 8, the evaluation path is indicated by a thick line, and the other paths are indicated by a broken line.

Here, the evaluation path is displayed to be distinguishable from other paths by changing the line type indicating the evaluation path, but this is merely an example. The evaluation path may be distinguishable from other paths by changing a color of the evaluation path, or the evaluation path may be distinguishable from other paths by surrounding the evaluation path with a frame line.

In addition, in the metabolism prediction image 64A, the compound image 66D selected via the pointer 54 has a thicker frame than before the selection. As a result, the metabolite D selected for displaying the toxicity determination flow 32E is distinguishable from the other compounds (for example, the test substance A, the metabolite B, and the metabolite C) of the prediction result. Changing the thickness of the frame line is merely an example, and, for example, an aspect may also be used, such as displaying a text such as "being selected" or making the frame line blink.

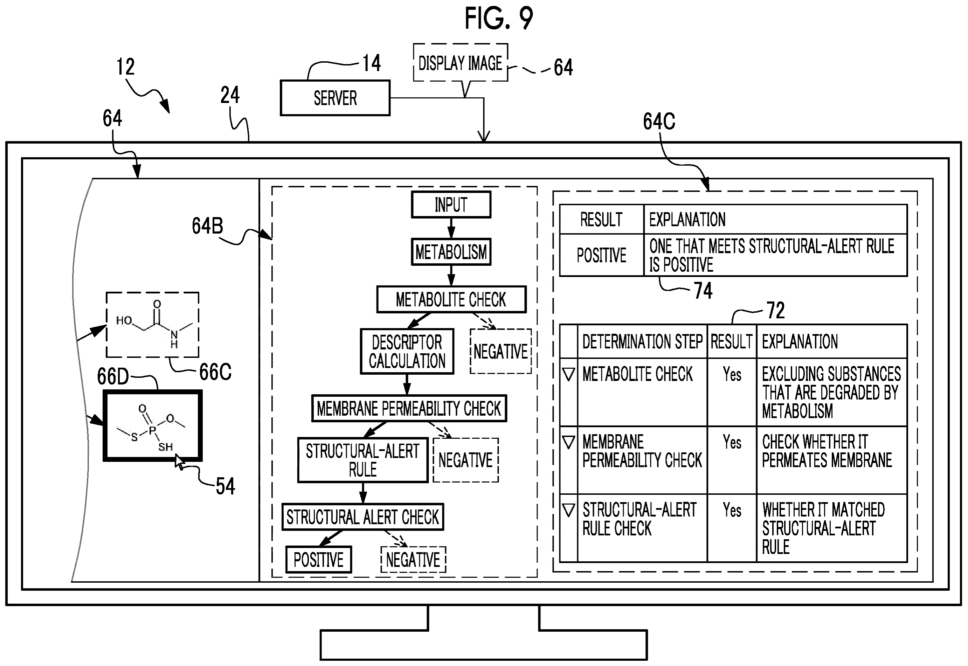

As shown in FIG. 9 as an example, in the display image 64, the flow explanation image 64C is displayed on a right side of the flow image 64B. As described above, the flow explanation image 64C includes the determination step explanation image 72 and the result explanation image 74. In a case where the compound image 66 showing a compound is selected in the metabolism prediction image 64A, an explanation of the determination step used in the toxicity evaluation for the selected compound is displayed in the flow explanation image 64C. In the example shown in FIG. 9, in a case where the compound image 66D is selected by the user 18 via the pointer 54, an explanation of the determination step of the toxicity evaluation for the metabolite D is displayed in the flow explanation image 64C.

In the example shown in FIG. 9, "metabolite check" is shown as the determination step, and an explanation of the determination content of "excluding substances that are degraded by metabolism" is shown as the corresponding explanation. In addition, "membrane permeability check" is shown as the determination step, and an explanation of the determination content of "check whether it permeates membrane" is shown as the corresponding explanation. Furthermore, "structural-alert rule check" is shown as the determination step, and an explanation of the determination content of "whether it matched structural-alert rule" is shown as the corresponding explanation.

In addition, in the example shown in FIG. 9, the result explanation image 74 is shown above the determination step explanation image 72. The result explanation image 74 shows "positive" as the final result of the toxicity evaluation, and shows an explanation of the final evaluation result that "one that meets structural-alert rule is positive".

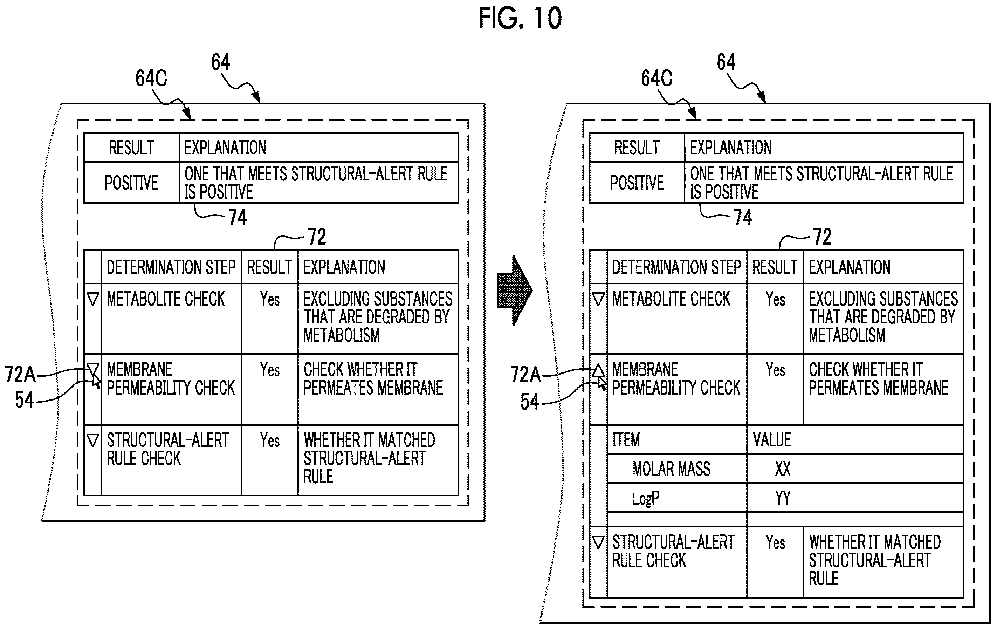

As shown in FIG. 10 as an example, in the determination step explanation image 72 of the flow explanation image 64C, a drop-down button 72A is shown for each determination step. In a case where the drop-down button 72A is clicked via the pointer 54, details of the determination result of the determination step are displayed. In the example shown in FIG. 10, in a case where the drop-down button 72A of the determination step of the membrane permeability check is clicked, sub-items are displayed as details of the determination result in the membrane permeability check. The details of the determination result include a numerical value used in a comparison with a threshold value in a case where the determination step involves the comparison with the threshold value, the numerical value indicating a characteristic of a molecular structure of the metabolite D. Here, "XX" is shown as a value of the molar mass of the metabolite D, and "YY" is shown as a value of the LogP of the metabolite D.

In the determination step explanation image 72 in which the details of the determination result are displayed, by clicking the drop-down button 72A again, the sub-items are collapsed. That is, the details of the determination result are not displayed. As described above, in the determination step explanation image 72, the display of the details of the determination result is switchable between display and non-display.

The user 18 can recognize the determination content of the determination step by visually recognizing the determination step explanation image 72. In a case where the details of the determination result are displayed in the determination step explanation image 72, the user 18 can check the details of the determination result of the determination step by visually recognizing the determination step explanation image 72.

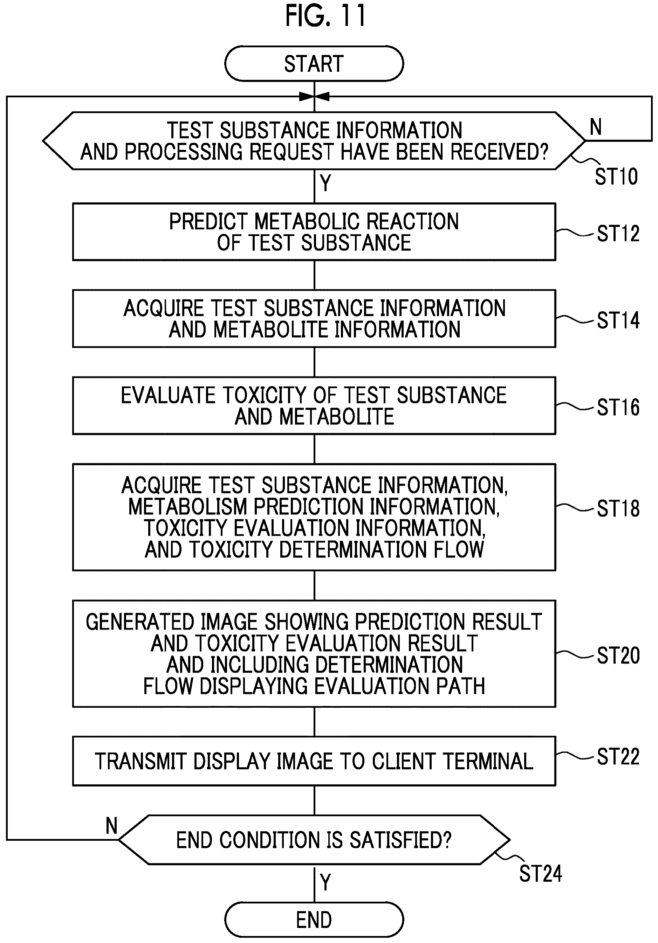

Next, control processing of the server 14 according to the present embodiment will be described with reference to FIG. 11. The server control processing is processing executed by the server 14, and includes the above-described metabolism prediction processing, toxicity evaluation processing, and image generation processing. FIG. 11 is a flowchart showing an example of the server control processing. The flow of the processing shown in FIG. 11 is an example of an "information processing method" according to the technology of the present disclosure.

In the server control processing shown in FIG. 11 as an example, first, in step ST10, the first acquisition unit 30A determines whether or not the test substance information 58 and the processing request have been acquired. In a case where it is determined in the determination in step ST10 that the test substance information 58 and the processing request have been acquired, the determination is affirmative, and the server control processing proceeds to step ST12. In a case where it is determined in the determination in step ST10 that the test substance information 58 and the processing request have not been acquired, the determination is negative, and the server control processing returns to step ST10.

In step ST12, the metabolism prediction unit 30B predicts a metabolic reaction based on the test substance information 58 acquired in step ST10. Specifically, the metabolism prediction unit 30B inputs the test substance information 58 to the metabolism prediction model 32D. The metabolism prediction model 32D outputs the metabolism prediction information 60 indicating a metabolite corresponding to the test substance. The metabolism prediction unit 30B acquires the metabolism prediction information 60. After the process of step ST12 is executed, the server control processing proceeds to step ST14.

In step ST14, the second acquisition unit 30C acquires the test substance information 58 and the metabolite information 60A. After the process of step ST14 is executed, the server control processing proceeds to step ST16.

In step ST16, the toxicity evaluation unit 30D evaluates the toxicity of the test substance indicated by the test substance information 58 acquired in step ST14 and the toxicity of the metabolite indicated by the metabolite information 60A. Specifically, the toxicity evaluation unit 30D executes the toxicity evaluation of the test substance and the metabolite by using the toxicity determination flow 32E. The toxicity evaluation unit 30D acquires the toxicity evaluation information 62 which is information indicating the result of the toxicity evaluation of the test substance and the metabolite. After the process of step ST16 is executed, the server control processing proceeds to step ST18.

In step ST18, the third acquisition unit 30E acquires the test substance information 58, the metabolism prediction information 60, the toxicity evaluation information 62, and the toxicity determination flow 32E. After the process of step ST18 is executed, the server control processing proceeds to step ST20.

In step ST20, the image generation unit 30F generates the display image 64 based on the test substance information 58, the metabolism prediction information 60, the toxicity evaluation information 62, and the toxicity determination flow 32E acquired in step ST18. The display image 64 is an image showing the prediction result and the evaluation result of the toxicity, and includes the flow image 64B in which the evaluation path is displayed. After the process of step ST20 is executed, the server control processing proceeds to step ST22.

In step ST22, the output unit 30G executes control of outputting the display image 64 generated by the image generation unit 30F in step ST20 to the client terminal 12. Specifically, the output unit 30G transmits the display image 64 to the client terminal 12. After the process of step ST22 is executed, the server control processing proceeds to step ST24.

In step ST24, the output unit 30G determines whether or not a condition (hereinafter, referred to as an "end condition") for ending the server control processing is satisfied. An example of the end condition is that the display image 64 has been transmitted to the client terminal 12. Another example of the end condition is that an instruction to end the server control processing has been accepted. In step ST24, in a case where the end condition is not satisfied, the determination is negative, and the server control processing proceeds to step ST10. In step ST24, in a case where the end condition is satisfied, the determination is affirmative, and the server control processing ends.

As described above, in the information processing system 10 according to the present embodiment, the display image 64 is generated by the processor 30 of the server 14. The display image 64 shows the prediction results of the metabolic reactions of the test substance A in the body and the metabolites B to D generated by the metabolic reactions, and the evaluation result of the toxicity of at least one compound in a compound group composed of the test substance A and the metabolites B to D. In addition, the display image 64 shows the toxicity determination flow 32E that is used in the evaluation of the toxicity and that has a plurality of determination steps. In the display image 64, in a case where a compound is selected from the prediction results, the evaluation path of the compound is displayed to be distinguishable from other paths. As a result, in the toxicity determination flow 32E, it is easier to understand which evaluation path has been followed for the toxicity evaluation. As a result, this configuration makes it possible to support the user in evaluating the toxicity of the compound.

For example, in the explanation of the evaluation result of the toxicity, in this configuration, it is easier to understand the evaluation of the toxicity since it is possible to see at a glance which evaluation path has been followed in the toxicity determination flow 32E, as compared with a case where the evaluation logic of the toxicity is described in a sentence.

In addition, in the information processing system 10 according to the present embodiment, the display image 64 includes the flow explanation image 64C in which an explanation regarding the toxicity determination flow 32E is displayed. In the flow explanation image 64C, an explanation of the determination content for each determination step is displayed. This makes it easier to understand what kind of determination is made in the determination step of the toxicity determination flow 32E.

In addition, in the information processing system 10 according to the present embodiment, in the flow explanation image 64C, the details of the determination result are displayed for each determination step included in the toxicity determination flow 32E. This makes it easier to understand what kind of determination result is obtained in the determination step of the toxicity determination flow 32E.

In addition, in the information processing system 10 according to the present embodiment, the details of the determination result shown in the flow explanation image 64C include a numerical value used in comparison with a threshold value in a case where the determination step involves the comparison with the threshold value, the numerical value indicating a characteristic of a molecular structure of the metabolite (for example, LogP or molar mass). This makes it easier to understand how the numerical value indicating the characteristic compares with the threshold value in a case where the determination step involves the comparison with the threshold value.

In addition, in the information processing system 10 according to the present embodiment, the display of the details of the determination result is switchable between display and non-display. As a result, since the display range is adjusted such that details of the determination result of the determination step in which a degree of interest of the user 18 is low are not displayed, it is easier to understand the determination step.

In addition, in the information processing system 10 according to the present embodiment, in the display image 64, the evaluation result of the toxicity of the compound is displayed in a distinguishable manner for each compound shown in the metabolism prediction image 64A. As a result, the evaluation result of the toxicity is displayed in a distinguishable manner. Therefore, for example, in a case where there is a compound predicted to be positive, by selecting the compound in the metabolism prediction image 64A, the evaluation path of the toxicity determination flow 32E is displayed in a distinguishable manner. This makes it easier for the user 18 to understand which evaluation path the compound of interest in terms of the evaluation result has followed.

In addition, in the information processing system 10 according to the present embodiment, in the display image 64, the compound selected for displaying the toxicity determination flow 32E is displayed in the metabolism prediction image 64A in a distinguishable manner. This makes it easier to understand which compound in the metabolism prediction image 64A is selected for displaying the toxicity determination flow 32E. That is, it becomes easier to understand a correspondence relationship between the evaluation path of the displayed toxicity determination flow 32E and the compounds in the prediction results.

In addition, in the information processing system 10 according to the present embodiment, in the metabolism prediction image 64A of the display image 64, a metabolic reaction pathway systematically shown using the metabolic reactions that occur in a chain starting from the test substance A and the resulting metabolites B to D is shown. As a result, by systematically displaying the metabolites B to D starting from the test substance A and also displaying the toxicity evaluation, it is possible to efficiently understand the prediction result of the metabolic reaction and the evaluation result of the toxicity of the compound involved in the metabolic reaction. For example, it is easier to understand which compound has toxicity in the metabolic reaction pathway, as compared with a case where the compounds are displayed in a list regardless of the reaction pathway.

In addition, the information processing system 10 according to the present embodiment, the metabolic reaction pathway shown in the metabolism prediction image 64A of the display image 64 is a tree diagram shown by the compound image 66 representing each compound of the test substance A and the metabolites B to D and the connecting lines 68 connecting the compound images 66 before and after the metabolic reaction. By presenting the metabolic reaction pathway as a tree diagram, it is easy to understand how the compound has changed during the metabolic reaction pathway. As a result, the metabolic reaction pathway becomes easier to understand.

The flow of the toxicity evaluation in the toxicity determination flow 32E shown in the present embodiment is merely an example. The flow of the toxicity evaluation in the toxicity determination flow 32E can be appropriately selected or changed by the user. In addition, the determination steps shown in the flow image 64B do not necessarily have to be all of the determination steps included in the toxicity determination flow 32E, and may be in a form in which some of the determination steps are displayed. For example, the determination step of interest of the user 18 may be displayed as the flow image 64B.

In addition, in the present embodiment, although an example of a form in which the result of the toxicity evaluation shown in the metabolism prediction image 64A is distinguishable by the type of the frame line of the compound image 66 showing the compound has been described, the technology of the present disclosure is not limited to this. The display aspect of the result of the toxicity evaluation need only be an aspect in which the user 18 can distinguish the result of the toxicity evaluation need, and, for example, a color (for example, red for positive, green for negative, and gray for disappearance compound), a shape (circular, triangular, or quadrangular), and/or a thickness of the frame line of the compound image 66 showing the compound may be changed. In addition, a text and/or a mark indicating the toxicity evaluation may be added to the compound image 66.

In addition, in the present embodiment, although an example of a form in which the result of the toxicity evaluation shown in the metabolism prediction image 64A is displayed for all compounds has been described, the technology of the present disclosure is not limited to this. For example, the result of the toxicity evaluation for some compounds (for example, the test substance A) does not need to be displayed.

In addition, in the present embodiment, although an example of a form in which the compounds are systematically displayed by being arranged from left to right in the metabolism prediction image 64A has been described, the technology of the present disclosure is not limited to this. In the metabolism prediction image 64A, the compounds may be systematically displayed from right to left, or from top to bottom.

In addition, in the present embodiment, although an example of a form in which the connecting line 68 is an arrow in the metabolism prediction image 64A has been described, the technology of the present disclosure is not limited to this. The connecting line 68 need only be a line that connects the compound images 66 showing the compounds before and after the reaction, and the connecting line 68 may be a straight line or a curved line.

In addition, in the present embodiment, although an example of a form in which the test substance A and the metabolites B to D are shown by the compound image 66 showing the compound graph in the metabolism prediction image 64A has been described, the technology of the present disclosure is not limited to this. In the metabolism prediction image 64A, the user 18 need only recognize the test substance A and the metabolites B to D, and an image showing a CAS number, a substance name, or a structural formula may be displayed instead of or together with the compound image 66.

### (First Modification Example)

In the above embodiment, although an example of a form in which the connecting line 68 is displayed in the metabolism prediction image 64A has been described, the technology of the present disclosure is not limited to this. In the present first modification example, in the metabolism prediction image 64A, a name of the metabolic reaction is displayed in association with the connecting line 68.

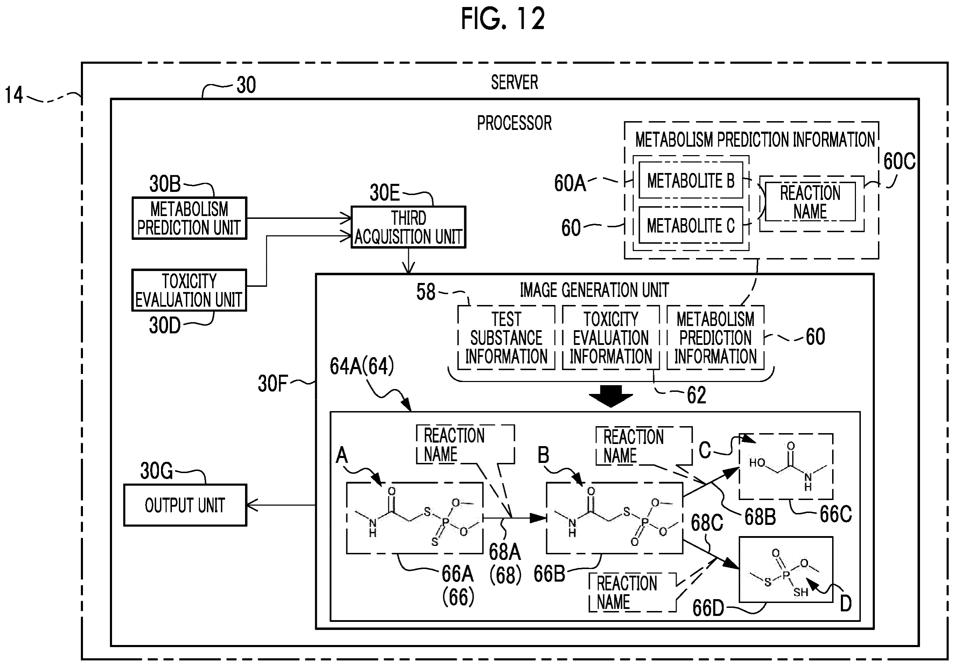

As shown in FIG. 12 as an example, in the processor 30 of the server 14, the third acquisition unit 30E acquires the test substance information 58, the metabolism prediction information 60, and the toxicity evaluation information 62. Here, the metabolism prediction information 60 includes the metabolite information 60A and reaction name information 60C. Here, the reaction name information 60C is information for specifying a name of the metabolic reaction. The reaction name information 60C is generated together with the metabolic reaction information 60B in the metabolism prediction processing. Then, the image generation unit 30F generates a display image 64 based on the test substance information 58, the metabolism prediction information 60, and the toxicity evaluation information 62. The display image 64 includes the metabolism prediction image 64A.

In the example shown in FIG. 12, the metabolism prediction image 64A includes a compound image 66 showing a compound graph of each of the test substance A and the metabolites B to D. In addition, the metabolism prediction image 64A includes a connecting line 68 that connects the compound images 66 before and after the metabolic reaction. A reaction name of the metabolic reaction is associated with the connecting line 68 based on the reaction name information 60C.

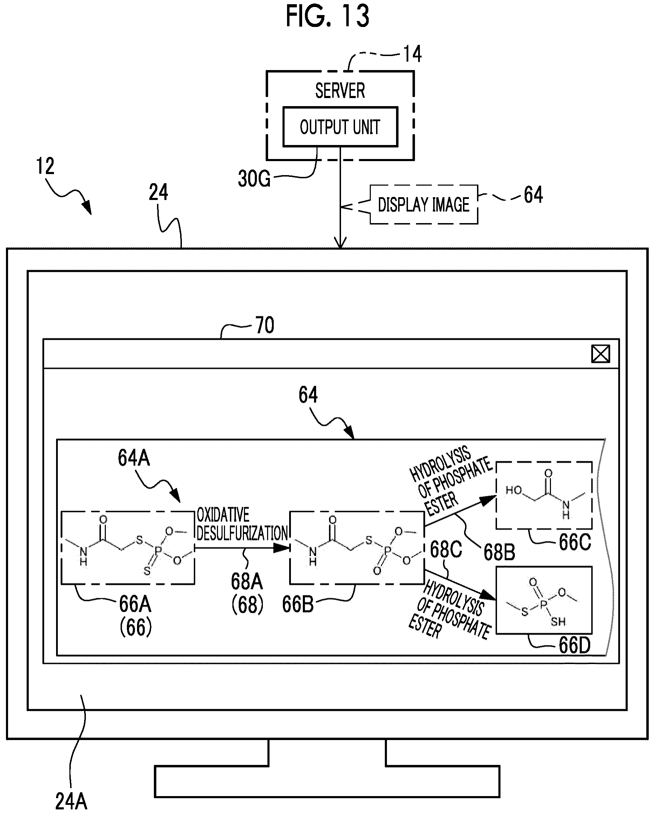

As shown in FIG. 13 as an example, the output unit 30G of the server 14 executes control of outputting the display image 64 to the client terminal 12. On the display device 24 of the client terminal 12, a screen 24A including the display image 64 is displayed.

In the example shown in FIG. 13, the metabolism prediction image 64A is displayed in the window 70. The metabolism prediction image 64A shows, as a tree diagram, the metabolic reaction pathway that systematically shows the metabolic reactions that occur in a chain starting from the test substance A and the resulting metabolites B to D, and shows the evaluation result of the toxicity of the compound in a distinguishable manner. In addition, in the metabolism prediction image 64A, a text indicating the reaction name corresponding to the connecting line 68 is displayed.

In the example shown in FIG. 13, in the metabolism prediction image 64A, a text "oxidative desulfurization" is displayed above the connecting line 68A. This indicates that test substance A is changed to the metabolite B by oxidative desulfurization. In addition, in the metabolism prediction image 64A, a text of "hydrolysis of phosphate ester" is displayed above the connecting line 68B. This indicates that the metabolite B is changed to the metabolite C by hydrolysis of the phosphate ester. In addition, in the metabolism prediction image 64A, a text of "hydrolysis of phosphate ester" is displayed below the connecting line 68C. This indicates that the metabolite B is changed to the metabolite D by hydrolysis of the phosphate ester. As described above, in the metabolism prediction image 64A, the connecting line 68 and the reaction name are displayed in association with each other.

The user 18 can understand the result of the metabolism prediction and the result of the toxicity evaluation by visually recognizing the metabolism prediction image 64A. In addition, the user 18 can recognize the name of the metabolic reaction by visually recognizing the metabolism prediction image 64A.

As described above, in the information processing system 10 according to the present first modification example, in the processor 30 of the server 14, the image generation unit 30F generates the metabolism prediction image 64A. The metabolism prediction image 64A includes a connecting line 68 that connects the compound images 66 before and after the metabolic reaction. Then, the name of the metabolic reaction is displayed in association with the connecting line 68. This makes it easier to understand what reactions have changed compounds during the metabolic reaction pathway. As a result, it becomes easier to understand the metabolic reaction pathway as compared with a case where the reaction name is not displayed.

In the first modification example, although an example of a form in which the reaction name is displayed in text in the metabolism prediction image 64A has been described, the technology of the present disclosure is not limited to this. It is sufficient that the user 18 can specify the metabolic reaction by visually recognizing the metabolism prediction image 64A, and, in the metabolism prediction image 64A, a mark indicating the reaction name may be displayed instead of or together with the text indicating the reaction name.

### [Second Embodiment]

In the first embodiment, although an example of a form in which the metabolic reaction pathway is shown by the tree diagram in the metabolism prediction image 64A has been described, the technology of the present disclosure is not limited to this. In the present second embodiment, in the metabolism prediction image 64A, the compound images 66 are displayed in a list.

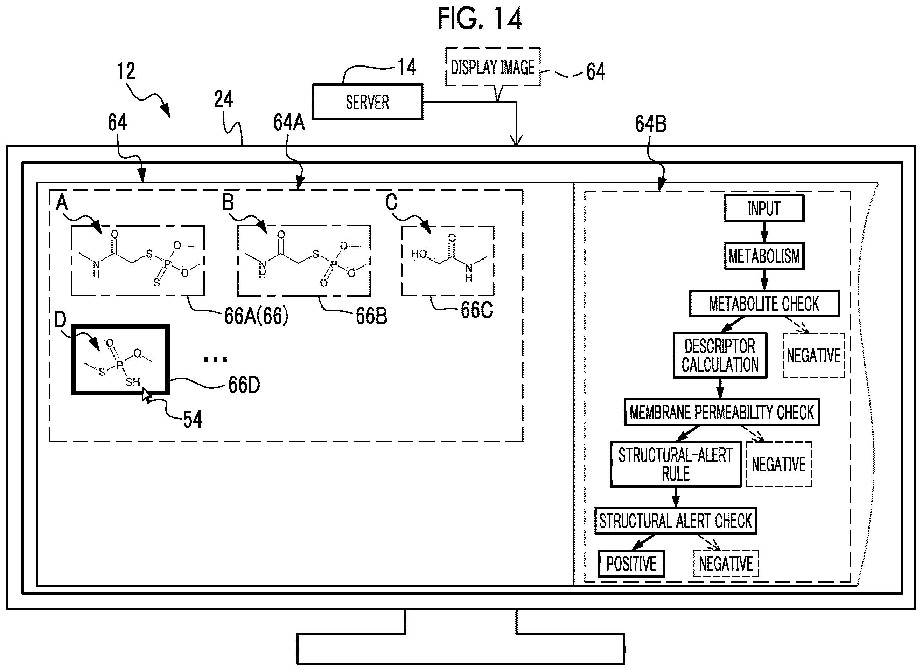

As shown in FIG. 14 as an example, the output unit 30G of the server 14 executes control of outputting the display image 64 to the client terminal 12. On the display device 24 of the client terminal 12, a screen 24A including the display image 64 is displayed.

In the metabolism prediction image 64A, the compound images 66 are displayed in a list. Specifically, the compound image 66A is shown at the upper left of a metabolism prediction image 64A, and, starting from the compound image 66A, the compound images 66B and 66C are displayed in this order along a right direction of the paper. Then, the compound image 66D is shown on a left side of a row below a row on which the compound images 66A to 66C are displayed. As described above, in the metabolism prediction image 64A, the compound images 66 are displayed in a list.

In addition, in the example shown in FIG. 14, in the display image 64, the flow image 64B is displayed on a right side of the metabolism prediction image 64A. In a case where the compound image 66 showing the compound is selected in the metabolism prediction image 64A, an evaluation path is displayed in the flow image 64B. In the example shown in FIG. 14, in a case where the compound image 66D is selected by the user 18 via the pointer 54, an evaluation path of the metabolite D indicated by the compound image 66D (here, a path leading to "positive") is displayed in the flow image 64B in a distinguishable manner from other paths.

As described above, in the information processing system 10 according to the present second embodiment, the display image 64 is generated by the processor 30 of the server 14. The display image 64 shows the prediction results of the metabolic reactions of the test substance A in the body and the metabolites B to D generated by the metabolic reactions, and the evaluation result of the toxicity of at least one compound in a compound group composed of the test substance A and the metabolites B to D. In addition, the display image 64 shows the toxicity determination flow 32E that is used in the evaluation of the toxicity and that has a plurality of determination steps. In the display image 64, in a case where a compound is selected from the prediction results, the evaluation path of the compound is displayed to be distinguishable from other paths. As a result, in the toxicity determination flow 32E, it is easier to understand which evaluation path has been followed for the toxicity evaluation.

### (Second Modification Example)

In each of the above-described embodiments, although an example of a form in which the metabolic reaction pathway is displayed as it is in the display image 64 has been described, the technology of the present disclosure is not limited to this. In the present second modification example, in the display image 64, the display range in the metabolism prediction image 64A, the display range in the flow image 64B, and the display range in the flow explanation image 64C can be adjusted.

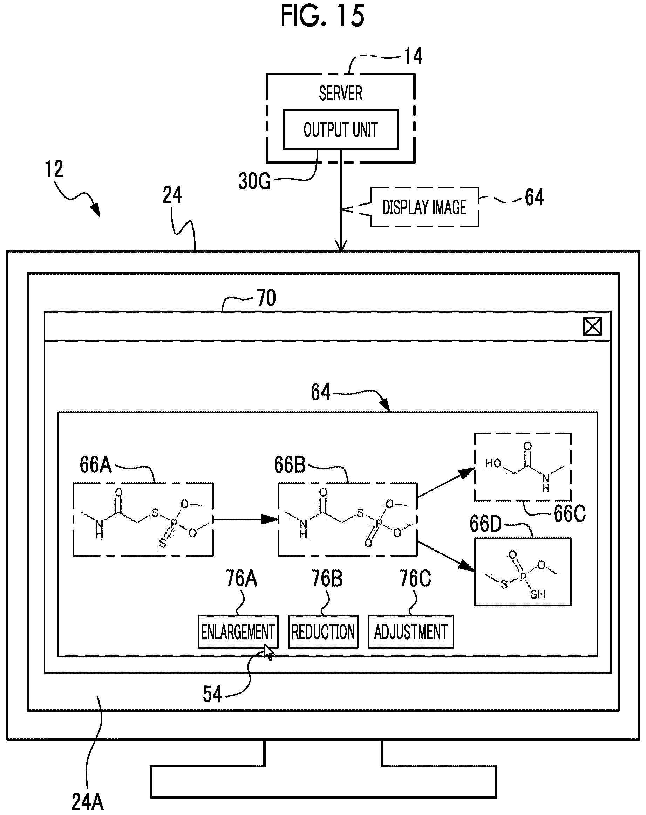

As shown in FIG. 15 as an example, an enlargement soft key 76A, a reduction soft key 76B, and an adjustment soft key 76C are displayed in a lower part of the metabolism prediction image 64A. Hereinafter, although adjustment of the display range in the metabolism prediction image 64A will be described, the same applies to the flow image 64B and the flow explanation image 64C.

The enlargement soft key 76A is a soft key for enlarging the display magnification of the metabolic reaction pathway, and the reduction soft key 76B is a soft key for reducing the display magnification of the metabolic reaction pathway. The user 18 presses the enlargement soft key 76A or the reduction soft key 76B via the pointer 54 to increase or decrease the display magnification of the metabolic reaction pathway. In addition, the adjustment soft key 76C is a soft key for restoring the display range of the metabolic reaction pathway to the original range. The user 18 presses the adjustment soft key 76C via the pointer 54 to return the display range of the metabolic reaction pathway to a state before the change in the display range. As described above, in the display image 64, the display range of the metabolic reaction pathway can be adjusted. By adjusting the display range of the metabolic reaction pathway, it becomes easier to understand the metabolic reaction pathway in a case where the metabolic reaction pathway is complex (for example, in a case where the metabolic reaction pathway branches multiple times and many metabolites are generated).

In the present second modification example, although an example of a form in which the display range is enlarged or reduced by the enlargement soft key 76A or the reduction soft key 76B has been described, the technology of the present disclosure is not limited to this. For example, in a case where the mouse 22 as the reception device 20 comprises a wheel, the display image 64 may be enlarged or reduced by operating the wheel.

### [Third Embodiment]

In the first embodiment, although an example of a form in which the metabolism prediction processing, the toxicity evaluation processing, and the image generation processing are performed in the server 14 has been described, the technology of the present disclosure is not limited to this. In the present third embodiment, the metabolism prediction processing and the toxicity evaluation processing are executed in the server 14, and the image generation processing is executed in the client terminal 12. In the present embodiment, the client terminal 12 is an example of an "information processing apparatus" according to the technology of the present disclosure.

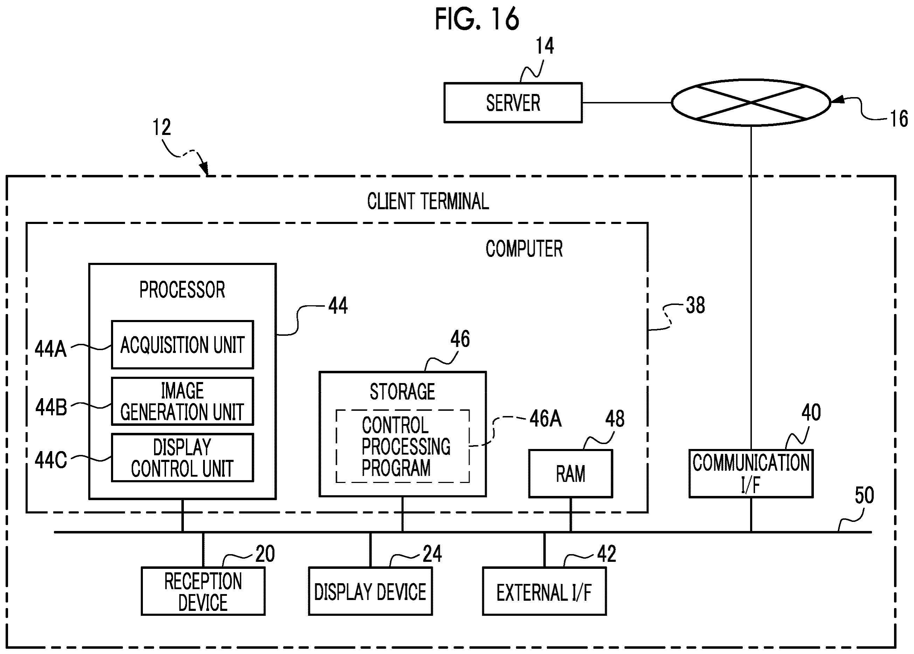

As shown in FIG. 16 as an example, the client terminal 12 comprises a computer 38, the reception device 20, the display device 24, a communication I/F 40, an external I/F 42, and a bus 50. The computer 38 comprises a processor 44, a storage 46, and a RAM 48. The processor 44, the storage 46, the RAM 48, the reception device 20, the display device 24, the communication I/F 40, and the external I/F 42 are connected to the bus 50. The processor 44 is an example of a "processor" according to the technology of the present disclosure.

In addition, since a hardware configuration (that is, the processor 44, the storage 46, and the RAM 48) of the computer 38 is basically the same as the hardware configuration of the computer 26, a description of the hardware configuration of the computer 38 will be omitted here.

The communication I/F 40 is connected to the network 16. The communication I/F 40 is responsible for transmitting and receiving information to and from an external communication device (for example, the server 14) via the network 16. For example, the communication I/F 40 transmits information in response to a request from the processor 44 to the external communication device via the network 16. In addition, the communication I/F 40 receives the information transmitted from the external communication device, and outputs the received information to the processor 44 via the bus 50.

The external I/F 42 is responsible for transmitting and receiving various types of information to and from an external device (not shown) present outside the client terminal 12. The external device may be, for example, at least one of a smart device, a personal computer, a server, a universal serial bus (USB) memory, a memory card, or a printer. An example of the external I/F 42 is a USB interface. The external device is connected directly or indirectly to the USB interface.

A control processing program 46A is stored in the storage 46. The control processing program 46A is a program for executing display control of an image. The processor 30 executes display control processing by reading out the control processing program 46A from the storage 46 and executing the read-out control processing program 46A on the RAM 48. The display control processing is realized by the processor 44 operating as an acquisition unit 44A, an image generation unit 44B, and a display control unit 44C. The computer 38 is an example of a "computer" according to the technology of the present disclosure, and the control processing program 46A is an example of a "program" according to the technology of the present disclosure.

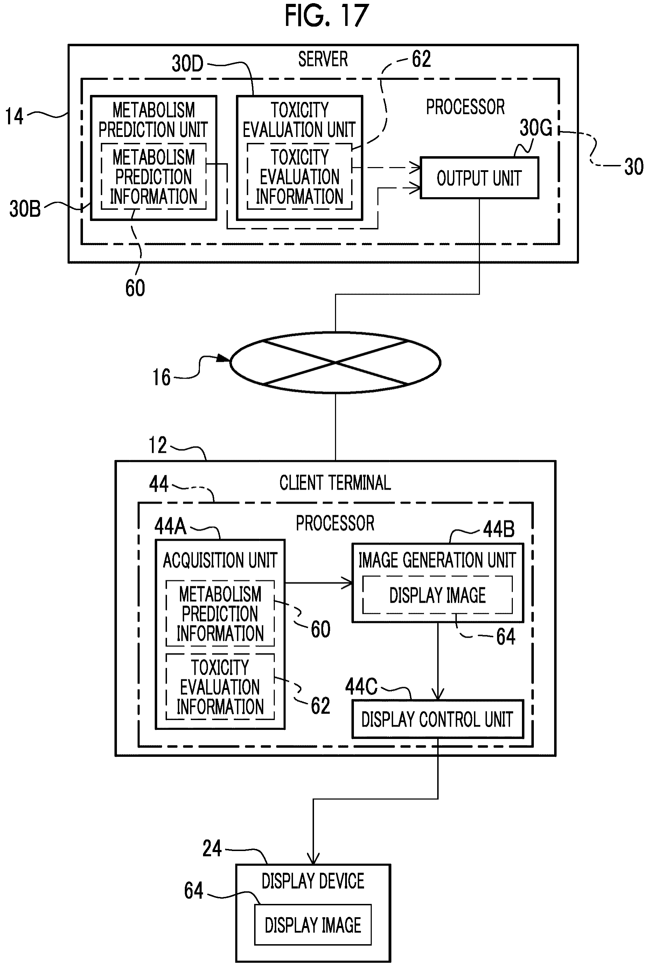

As shown in FIG. 17 as an example, in the processor 30 of the server 14, the metabolism prediction unit 30B outputs the metabolism prediction information 60 obtained by the metabolism prediction processing to the output unit 30G. In addition, the toxicity evaluation unit 30D outputs the toxicity evaluation information 62 obtained by the toxicity evaluation processing to the output unit 30G. The output unit 30G outputs the metabolism prediction information 60 and the toxicity evaluation information 62 to the client terminal 12 via the network 16.

In the processor 44 of the client terminal 12, the acquisition unit 44A acquires the metabolism prediction information 60 and the toxicity evaluation information 62 via the network 16. Then, the acquisition unit 44A outputs the metabolism prediction information 60 and the toxicity evaluation information 62 to the image generation unit 44B. The image generation unit 44B generates a display image 64 based on the metabolism prediction information 60, the toxicity evaluation information 62, and the toxicity determination flow 32E (not shown). Then, the image generation unit 44B outputs the generated display image 64 to the display control unit 44C. The display control unit 44C performs graphical user interface (GUI) control for displaying the display image 64, thereby causing the display device 24 to display the display image 64.

### [Fourth Embodiment]

In the first embodiment, although an example of a form in which the metabolism prediction processing, the toxicity evaluation processing, and the image generation processing are performed in the server 14 has been described, the technology of the present disclosure is not limited to this. In the present fourth embodiment, the metabolism prediction processing, the toxicity evaluation processing, and the image generation processing are performed in the client terminal 12. In the present embodiment, the client terminal 12 is an example of an "information processing apparatus" according to the technology of the present disclosure.

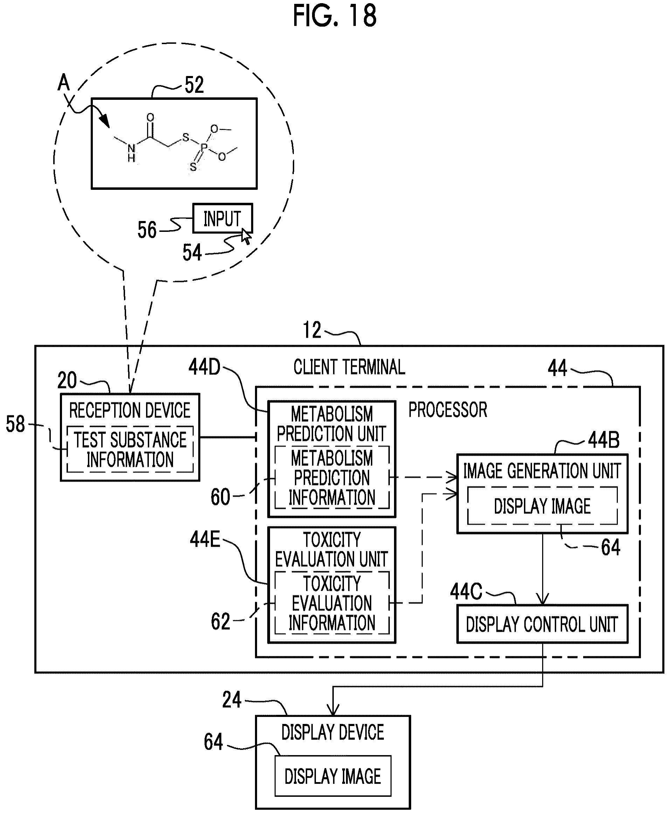

As shown in FIG. 18 as an example, the test substance information 58 is received in the client terminal 12 via the reception device 20. In the processor 44 of the client terminal 12, a metabolism prediction unit 44D performs the metabolism prediction of the test substance A based on the test substance information 58. In addition, a toxicity evaluation unit 44E performs the toxicity evaluation on the test substance A and the metabolites B to D. Then, the image generation unit 44B generates a display image 64 based on the metabolism prediction information 60, the toxicity evaluation information 62, and the toxicity determination flow 32E (not shown). The display control unit 44C causes the display device 24 to display the display image 64.

In addition, in each of the above-described embodiments, although an example of a form in which the metabolism prediction information 60 and the toxicity evaluation information 62 are obtained by executing the metabolism prediction processing and the toxicity evaluation processing has been described, the technology of the present disclosure is not limited to this. For example, the server 14 or the client terminal 12 may receive already obtained metabolism prediction information 60 and toxicity evaluation information 62 (for example, metabolism prediction information 60 and toxicity evaluation information 62 obtained as a result of processing by an external device, or metabolism prediction information 60 and toxicity evaluation information 62 obtained in the past), and perform the image generation processing.

In addition, in each of the above-described embodiments, although an example of a form in which the prediction processing program 32A, the evaluation processing program 32B, and the generation processing program 32C are stored in the storage 32 has been described, the technology of the present disclosure is not limited to this. For example, the prediction processing program 32A, the evaluation processing program 32B, and the generation processing program 32C may be stored in a portable storage medium such as an SSD or a USB memory. The storage medium is a non-transitory computer-readable storage medium. The prediction processing program 32A, the evaluation processing program 32B, and the generation processing program 32C stored in the storage medium are installed in the computer 26. The processor 30 executes control processing of the server 14 in accordance with the prediction processing program 32A, the evaluation processing program 32B, and the generation processing program 32C. Similarly, the control processing program 46A may be stored in a storage medium instead of the storage 46.

In each of the above-described embodiments, the computers 26 and 38 are exemplified, but the technology of the present disclosure is not limited to this, and a device including an application specific integrated circuit (ASIC), a field programmable gate array (FPGA), and/or a programmable logic device (PLD) may be applied instead of the computers 26 and 38. In addition, instead of the computers 26 and 38, a hardware configuration and a software configuration may be used in combination.

The following various processors can be used as hardware resources for executing the various kinds of processing described in each of the above-described embodiments. Examples of the processor include a CPU that is a general-purpose processor that functions as a hardware resource for executing the software, that is, the program to execute the metabolism prediction processing, the toxicity evaluation processing, and/or the image generation processing (hereinafter, also simply referred to as "various kinds of processing"). Examples of the processor also include a dedicated electronic circuit that is a processor whose dedicated circuit configuration is specially designed to execute specific processing, such as an FPGA, a PLD, or an ASIC. Any processor includes a memory built therein or connected thereto, and any processor uses the memory to execute various kinds of processing.

The hardware resource for executing the various kinds of processing may be configured by one of the various processors or by a combination of two or more processors of the same type or different types (for example, a combination of a plurality of FPGAs or a combination of a processor and an FPGA). Further, the hardware resource for executing the various kinds of processing may be one processor.

A first example of the configuration in which the hardware resource is configured by one processor is an aspect in which one processor is configured by a combination of one or more processors and software, and this processor functions as the hardware resource for executing the various kinds of processing. Second, as represented by system-on-a-chip (SoC), there is a form in which a processor that realizes the functions of the entire system including a plurality of hardware resources for executing the various kinds of processing with a single integrated circuit (IC) chip. As described above, the various kinds of processing are implemented by using one or more of the various processors as the hardware resource.

Further, specifically, an electronic circuit in which circuit elements, such as semiconductor elements, are combined can be used as the hardware structure of these various processors. The various kinds of processing are merely examples. Accordingly, it is possible to delete an unnecessary step, add a new step, or change a processing order without departing from the gist of the present disclosure.

The content of the above description and the content of the drawings are detailed explanations of the parts relating to the technology of the present disclosure, and are merely examples of the technology of the present disclosure. For example, description related to the above configurations, functions, actions, and effects is description related to an example of configurations, functions, actions, and effects of the parts relating to the technology of the present disclosure. Thus, it is needless to say that unnecessary portions may be deleted, new elements may be added, or replacement may be made to the content of the above description and the content of the drawings without departing from the gist of the technology of the present disclosure. In order to avoid complication and easily understand the parts relating to the technology of the present disclosure, in the content of the above description and the content of the drawings, the description regarding common general technical knowledge which is not necessarily particularly described in terms of embodying the technology of the present disclosure is omitted.

In the present specification, "A and/or B" is synonymous with "at least one of A or B". That is, "A and/or B" may refer to A alone, B alone, or a combination of A and B. In addition, in the present specification, in a case in which three or more matters are expressed with the connection of "and/or", the same concept as "A and/or B" is applied.

All documents, patent applications, and technical standards mentioned in the present specification are incorporated herein by reference to the same extent as in a case in which each document, each patent application, and each technical standard are specifically and individually described by being incorporated by reference.

The disclosure of JP2023-059423 filed on March 31, 2023 is incorporated herein by reference in its entirety.

In regard to the embodiments described above, the following appendices will be further disclosed.

### <Appendix 1>

An information processing apparatus comprising:
a processor,
in which the processor is configured to
   generate a display image that shows a prediction result of metabolic reactions occurring in vivo from a test substance and metabolites generated by the metabolic reactions and an evaluation result of toxicity of at least one compound in a compound group composed of the test substance and the metabolites, and that includes a determination flow indicating a toxicity determination procedure, which is used in an evaluation of the toxicity, has at least one determination step, and has a path that branches according to a determination result of the determination step, in which, in a case where the compound is selected in the display image, a path that leads to the evaluation result of the compound among a plurality of paths is shown in a distinguishable manner from the other paths, and
   execute control of outputting the generated display image.

### <Appendix 2>

The information processing apparatus according to Appendix 1,
in which the display image includes a first display region in which an explanation regarding the determination flow is displayed, and
an explanation of a determination content is displayed in the first display region for each determination step included in the determination flow.

### <Appendix 3>

The information processing apparatus according to Appendix 2,
in which, in the first display region, details of the determination result are displayed for each determination step included in the determination flow.

### <Appendix 4>

The information processing apparatus according to Appendix 3,
in which the details of the determination result include a numerical value used in comparison with a threshold value in a case where the determination step involves the comparison with the threshold value, the numerical value indicating a characteristic of a molecular structure of the metabolite.

### <Appendix 5>

The information processing apparatus according to Appendix 3 or 4,
in which the details of the determination result is switchable between display and non-display.

### <Appendix 6>

The information processing apparatus according to any one of Appendices 1 to 5,
in which, in the display image, the evaluation result of the compound is displayed in a distinguishable manner for each compound in the prediction result.

### <Appendix 7>

The information processing apparatus according to any one of Appendices 1 to 6,
in which, in the display image, the compound selected for displaying the determination flow is displayed in a distinguishable manner in the prediction result.

### <Appendix 8>

The information processing apparatus according to any one of Appendices 1 to 8,
in which, in the display image, as the prediction result, a metabolic reaction pathway that is systematically shown using the metabolic reactions occurring in a chain starting from the test substance and the resulting metabolites is shown.

### <Appendix 9>

The information processing apparatus according to Appendix 8,
in which the metabolic reaction pathway shown in the display image is a tree diagram shown by compound data representing each compound of the test substance and the metabolites and connecting lines connecting items of the compound data before and after the metabolic reaction.

### <Appendix 10>

The information processing apparatus according to Appendix 9,
in which a name of the metabolic reaction is displayed in association with the connecting line.

## Claims

1. An information processing apparatus comprising:
a processor,
wherein the processor is configured to
generate a display image that shows a prediction result of metabolic reactions occurring in vivo from a test substance and metabolites generated by the metabolic reactions and an evaluation result of toxicity of at least one compound in a compound group composed of the test substance and the metabolites, and that includes a determination flow indicating a toxicity determination procedure, which is used in an evaluation of the toxicity, has at least one determination step, and has a path that branches according to a determination result of the determination step, in which, in a case where the compound is selected in the display image, a path that leads to the evaluation result of the compound among a plurality of paths is shown in a distinguishable manner from the other paths, and
execute control of outputting the generated display image.

2. The information processing apparatus according to claim 1,
wherein the display image includes a first display region in which an explanation regarding the determination flow is displayed, and
an explanation of a determination content is displayed in the first display region for each determination step included in the determination flow.

3. The information processing apparatus according to claim 2,
wherein, in the first display region, details of the determination result are displayed for each determination step included in the determination flow.

4. The information processing apparatus according to claim 3,
wherein the details of the determination result include a numerical value used in comparison with a threshold value in a case where the determination step involves the comparison with the threshold value, the numerical value indicating a characteristic of a molecular structure of the metabolite.

5. The information processing apparatus according to claim 4,
wherein the details of the determination result are switchable between display and non-display.

6. The information processing apparatus according to claim 1,
wherein, in the display image, the evaluation result of the compound is displayed in a distinguishable manner for each compound in the prediction result.

7. The information processing apparatus according to claim 1,
wherein, in the display image, the compound selected for displaying the determination flow is displayed in a distinguishable manner in the prediction result.

8. The information processing apparatus according to claim 1,
wherein, in the display image, as the prediction result, a metabolic reaction pathway that is systematically shown using the metabolic reactions occurring in a chain starting from the test substance and the resulting metabolites is shown.

9. The information processing apparatus according to claim 8,
wherein the metabolic reaction pathway shown in the display image is a tree diagram shown by compound data representing each compound of the test substance and the metabolites and connecting lines connecting items of the compound data before and after the metabolic reaction.

10. The information processing apparatus according to claim 9,
wherein a name of the metabolic reaction is displayed in association with the connecting line.

11. An information processing method comprising:
generating a display image that shows a prediction result of metabolic reactions occurring in vivo from a test substance and metabolites generated by the metabolic reactions and an evaluation result of toxicity of at least one compound in a compound group composed of the test substance and the metabolites, and that includes a determination flow indicating a toxicity determination procedure, which is used in an evaluation of the toxicity, has at least one determination step, and has a path that branches according to a determination result of the determination step, in which, in a case where the compound is selected in the display image, a path that leads to the evaluation result of the compound among a plurality of paths is shown in a distinguishable manner from the other paths; and
executing control of outputting the generated display image.

12. A program for causing a computer to execute a process comprising:
generating a display image that shows a prediction result of metabolic reactions occurring in vivo from a test substance and metabolites generated by the metabolic reactions and an evaluation result of toxicity of at least one compound in a compound group composed of the test substance and the metabolites, and that includes a determination flow indicating a toxicity determination procedure, which is used in an evaluation of the toxicity, has at least one determination step, and has a path that branches according to a determination result of the determination step, in which, in a case where the compound is selected in the display image, a path that leads to the evaluation result of the compound among a plurality of paths is shown in a distinguishable manner from the other paths; and
executing control of outputting the generated display image.
